# EUROPÄISCHE PATENTSCHRIFT

(11) **EP 2 707 351 B1**
(45) Veröffentlichungstag und Bekanntmachung des Hinweises auf die Patenterteilung: **06.11.2019**
(21) Anmeldenummer: 12719976.8
(22) Anmeldetag: 09.05.2012
(51) Int. Cl.: C07C 29/145, C07C 29/17, C07C 29/80

(54) **VERFAHREN ZUR HERSTELLUNG VON 1,2 PENTANDIOL**
METHOD FOR PRODUCING 1-2-PENTANEDIOL
PROCÉDÉ DE PRÉPARATION DE PENTANE-1,2-DIOL

(30) Priorität: 09.05.2011 US 201161483914 P
(43) Veröffentlichungstag der Anmeldung: 19.03.2014
(73) Patentinhaber: Symrise AG, 37603 Holzminden (DE)
(72) Erfinder: KOCH, Oskar, 37079 Göttingen (DE); KÖCKRITZ, Angela, 12437 Berlin (DE); KANT, Michael, 12693 Berlin (DE); MARTIN, Andreas, 12524 Berlin (DE); SCHÖNING, Axel, 37603 Holzminden (DE); ARMBRUSTER, Udo, 18055 Rostok (DE); BARTOSZEK, Michael, 12683 Berlin (DE); EVERT, Sigrid, 15806 Zossen (DE); LANGE, Brigitte, 13055 Berlin (DE); BIENERT, Regina, 18059 Rostok (DE)
(74) Vertreter: Eisenführ Speiser
(86) Internationale Anmeldenummer: PCT/EP2012/058586
(87) Internationale Veröffentlichungsnummer: WO 2012/152849

(56) Entgegenhaltungen:
- WO-A2-2008/151269
- DE-C- 555 405
- DE-C- 746 307
- DE-C- 827 804
- US-A- 2 201 347
- WENJIE XU ET.AL.: "Direct catalytic conversion of furfural to 1,5-pentanediol by hydrogenolysis of the furan ring under mild conditions over Pt/Co2AlO4 catalyst", CHEM. COMMUN., Bd. 47, 23. Februar 2011 (2011-02-23), Seiten 3924-3926, XP002681491,

## Beschreibung

Die vorliegende Erfindung betrifft ein Verfahren zur Herstellung von 1,2-Pentandiol (1,2-n-Pentandiol) durch Umsetzung eines Edukts umfassend eine oder beide Verbindungen aus der Gruppe bestehend aus Furfurylalkohol und Furfural in Gegenwart eines ersten heterogenen Katalysators mit Wasserstoff. Solche Verfahren umfassen auch Verfahren zur Herstellung von 1,2-Pentandiol (1,2-n-Pentandiol) aus Furfurylalkohol und Wasserstoff in Gegenwart eines heterogenen Platinkatalysators umfassend (i) Platin und/oder eine oder mehrere Platinverbindungen und (ii) ein oder mehrere Trägermaterialien.

Im Bereich der Kosmetik wird 1,2-Pentandiol beispielsweise unter anderem als Hautbefeuchtungsmittel (EP 0 655 904) oder als antimikrobielles Mittel (EP 1 478 231) eingesetzt.

1,2-Pentandiol schützt die Haut vor umwelt- und witterungsbedingter Austrocknung und ist ein wichtiger Wirkstoff in kosmetischen Formulierungen.

Eine der Anforderungen an die Qualität des 1,2-Pentandiols, insbesondere für den Einsatz im Bereich der Kosmetik, ist ein möglichst neutraler Geruch. Ein unangenehmer oder störender Geruch wird dabei durch die das 1,2-Pentandiol vergesellschaftenden anderen Substanzen hervorgerufen, da 1,2-Pentandiol in reiner Form weitgehend bzw. vollkommen geruchlos ist.

Je nach Herstellungsverfahren enthält das jeweils erhaltene Rohprodukt neben dem gewünschten 1,2-Pentandiol verschiedene Nebenprodukte, die mehr oder weniger stark und teilweise sehr unangenehm riechen.

Diese nicht erwünschten riechenden Verbindungen lassen sich im Rahmen eines sich anschließenden Reinigungsverfahrens teilweise nur in unzureichendem Maße oder durch aufwendige zusätzliche Verfahrensschritte entfernen bzw. vermeiden.

US 6,528,665 B1 schlägt ein Verfahren zur Herstellung möglichst reiner Alkandiole vor. Dabei wird gemäß US 6,528,665 B1 eine Reinigung auf der Stufe der Epoxyalkane durchgeführt, bevor diese zu den entsprechenden Alkandiolen hydrolysiert werden.

EP 1 876 162 A1 beschreibt die Herstellung von Alkandiolen aus den entsprechenden Olefinen mittels Epoxidation und anschließender Hydrolyse. Die so erhaltenen Rohprodukte wurden dort mittels sich anschließender Behandlung weiter gereinigt, und unangenehm riechende Nebenprodukte zu entfernen.

Die Herstellung von 1,2-Pentandiol erfolgt heute regelmäßig aus n-Pent-1-en, welches aus petrochemischen Quellen (noch) verfügbar ist. Das n-Pent-1-en wird mit Hilfe von Peroxiden (z.B. Wasserstoffperoxid) zum entsprechenden Epoxid umgesetzt und anschließend mit organischen Säuren wie Ameisensäure oder Mineralsäuren wie Schwefelsäure in 1,2-Pentandiol überführt.

Diese Herstellungsmethode ist in EP 0 257 243 bzw. EP 0 141 775 beschrieben und weist ökonomische und ökologische Nachteile auf. So muss der bei diesem Verfahren als Zwischenprodukt entstehende Diester des 1,2-Pentandiols verseift werden, um das 1,2-Pentandiol zu erhalten. Wird die Epoxidation von n-Pent-1-en beispielsweise mit Wasserstoffperoxid und Ameisensäure durchgeführt, entsteht bei der anschließenden Verseifung des Diformiats des 1,2-Pentandiols mit Natronlauge Natriumformiat als Koppelprodukt, welches entsorgt werden muss und so beispielsweise das Abwasser belastet. Ferner hat n-Pent-1-en einen sehr niedrigen Siedepunkt, was bei Umgang mit und Lagerung von n-Pent-1-en wegen der bestehenden Explosionsgefahr besondere und aufwendigere Schutzmaßnahmen erfordert. Zudem wäre es wünschenswert einen einfachen und im technischen Maßstab praktikablen Syntheseweg zu finden, vorzugsweise ohne dabei einen petrochemischen Rohstoff einzusetzen.

Eine aus nachwachsenden Rohstoffen erhältliche Substanz ist Furfurylalkohol. Furfurylalkohol kann beispielsweise aus zuckerhaltigen Getreideabfällen in großen Mengen gewonnen werden.

Aus der Literatur ist bekannt, dass bei der Hydrierung bzw. Hydrogenolyse von Furfurylalkohol unterschiedliche Verbindungen entstehen.

So berichten Adkins und Connor [Journal of American Chemical Society 53, 1091 (1931)], dass bei der Hydrierung bzw. Hydrogenolyse von Furfurylalkohol bei 175 °C in flüssiger Phase unter Einsatz von Kupferchromit als Katalysator ein Gemisch aus 40% 1,2-Pentandiol, 30% 1,5-Pentandiol, 10% Amylalkohol sowie 20% an Tetrahydrofurfurylalkohol und Methyltetrahydrofuran entsteht. Kaufmann und Adams [Journal of American Chemical Society 45, 3029 (1923)] berichten, dass die Hydrogenolyse/Hydrierung von Furfural in Gegenwart von Platinmohr bei Raumtemperatur zu einem Gemisch von Furfurylalkohol, 1-Pentanol, Tetrahydrofurfurylalkohol, 1,2-Pentandiol und 1,5-Pentandiol führt.

Ferner findet man in den Arbeiten von Smith und Fuzek [Journal of American Chemical Society 71, 415 (1949)] Untersuchungen über katalytische Hydrierungen bzw. Hydrogenolysen von Furan und Furanderivaten in der Flüssigphase mittels Platindioxid-Katalysatoren. Die Reaktionen wurden in Essigsäure bei einem Wasserstoffdruck von 20, 40 bzw. 60 psi (60 psi entsprechen etwa 4 bar) durchgeführt, der genannte Katalysator wurde gemäß Literatur [Organic Synthesis 8, 92 (1928)] hergestellt. Bei der Hydrierung bzw. Hydrogenolyse von Furfurylalkohol unter Verwendung von Platindioxid als Katalysator soll angeblich in nahezu quantitativer Ausbeute 1,2-Pentandiol entstehen, welches dort als Diacetat von der Essigsäure abgetrennt wurde.

Des Weiteren beschreibt Xu [Chemical Communications 47, 3924 (2011)] die direkte Umsetzung von Furfural zu 1,5-Pentandiol mit hoher Ausbeute über einen durch Co-Präzipitation hergestellten Pt/Co₂AlO₄ Katalysator und einem Wasserstoffdruck von 1,0 bis 2,5 MPa (2,5 MPa entsprechen 25 bar). Dabei entsteht neben anderen Reaktionsprodukten auch 1,2-Pentandiol, jedoch nur in geringen Mengen.

In WO 2008/151269 wird die Umsetzung von Furfural mit Wasserstoff über einem Ruthenium enthaltenden sowie über einem Palladium enthaltenden Katalysator beschrieben. Die Umsetzung erfolgt dabei unter einem Wasserstoffdruck von 13,8 MPa (13,8 MPa entsprechen 138 bar) und erzeugt ebenfalls geringe Mengen an 1,2-Pentandiol.

Obwohl in eigenen Untersuchungen diese Arbeitsvorschrift zur Herstellung von 1,2-Pentandiol mehrfach nachgearbeitet wurde, konnte jedoch lediglich ein Produktgemisch aus Tetrahydrofurfurylalkohol, 2-Methyltetrahydrofuran, 1-Pentanol, 2-Pentanol, 1,2-Pentandiol und 1,5-Pentandiol erhalten werden, wobei die Ausbeute an 1,2-Pentandiol bei maximal 20% der Theorie lag.

Es bestand somit die Aufgabe, ausgehend von Furfurylalkohol und/oder Furfural ein möglichst effektives und/oder effizientes sowie vorzugsweise umweltfreundliches und/oder ressourcenschonendes Verfahren zur Herstellung von 1,2-Pentandiol zu entwickeln.

Es wurde nun gefunden, dass die Umsetzung eines Edukts umfassend eine oder beide Verbindungen aus der Gruppe bestehend aus Furfurylalkohol und Furfural mit Wasserstoff in Gegenwart eines heterogenen Katalysators umfassend
(i) ein oder mehrere Metalle aus der Gruppe bestehend aus Platin, Rhodium, Nickel und Iridium in metallischer Form und/oder eine oder mehrere Verbindungen von Metallen aus der Gruppe bestehend aus Platin, Rhodium, Nickel und Iridium,
   sowie
(ii) ein oder mehrere Trägermaterialien
   das gewünschte 1,2-Pentandiol in guter Ausbeute und/oder guter Selektivität ergibt.

Es wurde insbesondere gefunden, dass die Hydrogenolyse von Furfurylalkohol in Gegenwart eines heterogenen Platin-Katalysators das gewünschte 1,2-Pentandiol in guter Ausbeute und/oder guter Selektivität ergibt, wenn der heterogene Platin-Katalysator (i) Platin und/oder eine oder mehrere Platinverbindungen und (ii) ein oder mehrere Trägermaterialien umfasst. Dies gilt insbesondere, wenn die Hydrogenolyse in bestimmten Verdünnungsmitteln durchgeführt wird.

Gegenstand der vorliegenden Erfindung ist daher ein Verfahren zum Herstellen von 1,2-Pentandiol, umfassend den Schritt
- Umsetzung eines Edukts umfassend eine oder beide Verbindungen aus der Gruppe bestehend aus Furfurylalkohol und Furfural in Gegenwart eines ersten heterogenen Katalysators mit Wasserstoff unter Bildung eines Gemisches enthaltend 1,2-Pentandiol und gegebenenfalls eine oder mehrere Verbindungen aus der Gruppe bestehend aus 1-Hydroxy-2-pentanon, Furfurylalkohol und Furfural,
   wobei der erste heterogene Katalysator
   (i) ein oder mehrere Metalle aus der Gruppe bestehend aus Platin, Rhodium, Nickel, und Iridium in metallischer Form und/oder eine oder mehrere Verbindungen von Metallen aus der Gruppe bestehend aus Platin, Rhodium, Nickel, und Iridium, sowie
   (ii) ein oder mehrere Trägermaterialien
      umfasst.

Bevorzugt umfasst der erste heterogene Katalysator (i) Platin in metallischer Form und/oder eine oder mehrere Platin(IV)-Verbindungen. Besonders bevorzugt ist der erste heterogene Katalysator ausgewählt aus der Gruppe bestehend aus
- Katalysatoren umfassend (i) Platin in metallischer Form und (ii) Aktivkohle, insbesondere Platin auf Aktivkohle,
- Katalysatoren umfassend (i) Platin in metallischer Form und (ii) Aluminiumoxid, insbesondere Platin auf Aluminiumoxid,
- Katalysatoren umfassend (i) Platin in metallischer Form und (ii) Siliciumdioxid, insbesondere Platin auf Siliciumdioxid,
- Katalysatoren umfassend (i) Platin in metallischer Form und (ii) Siliciumcarbid, insbesondere Platin auf Siliciumcarbid,
- Katalysatoren umfassend (i) Platin(IV)-oxid und (ii) Aluminiumoxid, insbesondere Platindioxid auf Aluminiumoxid.

Vorzugsweise handelt es sich bei den erfindungsgemäß einzusetzenden ersten heterogenen Katalysatoren nicht um Katalysatoren, die neben einem Trägermaterial auch zwei Ingredienzien aufweisen und ausgewählt sind aus der folgenden Liste:
Aktivkohle / Pt / ReO₂,
MgO / Pd / MnO₂,
Al₂O₃/ Rh / MoO₃,
BaO / Ru / Co₃O₄,
Amorphes Aluminosilikat / Co / TiO₂,
SiO₂/ Ni / WO₃,
CeO / PtRh / Cr₂O₃,
CaO / NiPd / Fe₃O₄,
MgO-Al₂O₃/ RuFe / V₂O₅,
MgO / Pt / ReO₂,
Al₂O₃/ Pd / MnO₂,
BaO / Rh / MoO₃,
Amorphes Aluminosilikat / Ru / Co₃O₄,
SiO₂/ Co / TiO₂,
CeO / Ni / WO₃,
CoO / Pd / Cr₂O₃,
Fe₂O₃/ Rh / Fe₃O₄,
MnO₂/ Ru / V₂O₅.

Vorzugsweise handelt es sich bei den erfindungsgemäß einzusetzenden ersten heterogenen Katalysatoren nicht um solche Katalysatoren, bei denen die nachfolgenden Paare von Ingredienzien vorliegen:
Pt / ReO₂, Pd / MnO₂, Rh / MoO₃, Ru / Co₃O₄, Co / TiO₂, Ni / WO₃, PtRh / Cr₂O₃, NiPd / Fe₃O₄, RuFe / V205, Pd / Cr203, Rh / Fe₃O₄, Ru / V₂O₅.

Besteht der Bestandteil (i) des ersten heterogenen Katalysators aus Platin, so ist der erste heterogene Katalysator bevorzugt frei von ReO₂ und frei von Cr₂O₃, bevorzugt frei von Rhenium und frei von Chrom. Bevorzugt gilt dies auch für erste heterogene Katalysatoren, deren Bestandteil (i) Platin enthält.

Besteht der Bestandteil (i) des ersten heterogenen Katalysators aus Rhodium. so ist der erste heterogene Katalysator bevorzugt frei von MoO₃ und frei von Fe₂O₃ und frei von Fe₃O₄ und frei von Cr₂O₃, bevorzugt frei von Molybdän und frei von Eisen und frei von Chrom.

Besteht der Bestandteil (i) des ersten heterogenen Katalysators aus Nickel, so ist der erste heterogene Katalysator bevorzugt frei von WO₃, und frei von Fe₃O₄, bevorzugt frei von Wolfram und Eisen. Bevorzugt gilt dies auch für erste heterogene Katalysatoren, deren Bestandteil (i) Nickel enthält.

Vorzugsweise ist ein erfindungsgemäß einzusetzender erster heterogener Katalysator frei von Oxiden der Elemente Re, Mo, Mn, Co, Ti, W, Cr, Fe, V und Ta.

Bevorzugt liegt in dem ersten heterogenen Katalysator die Gesamtkonzentration an (i) Platin, Rhodium, Nickel und Iridium in metallischer Form und in Form von in Verbindungen dieser Metalle enthaltenem Platin, Rhodium, Nickel und Iridium im Bereich von 0,1 Gew.-% bis 50 Gew.-%, bevorzugt von 0,5 Gew.-% bis 20 Gew.-%, weiter bevorzugt von 1 bis 10 Gew.-%, besonders bevorzugt von 1 Gew.-% bis 5 Gew.-%, bezogen auf das Gesamtgewicht aller Bestandteile des ersten heterogenen Katalysators.

Bevorzugt liegt die Gesamtkonzentration an (i) Platin, Rhodium, Nickel und Iridium in metallischer Form und in Form von in Verbindungen dieser Metalle enthaltenem Platin, Rhodium, Nickel und Iridium im ersten heterogenen Katalysator im Bereich von 0,01 bis 10 Mol.-%, bevorzugt im Bereich von 0,05 bis 5 Mol.-%, weiter bevorzugt im Bereich von 0,1 bis 2 Mol.-%, bezogen auf die eingesetzte Gesamtstoffmenge an Furfurylalkohol und Furfural.

Bevorzugt liegt sowohl die auf das Gesamtgewicht aller Bestandteile des ersten heterogenen Katalysators bezogene Gesamtkonzentration an (i) Platin, Rhodium, Nickel und Iridium in metallischer Form und in Form von in Verbindungen dieser Metalle enthaltenem Platin, Rhodium, Nickel und Iridium als auch die auf die eingesetzte Gesamtstoffmenge an Furfurylalkohol und Furfural bezogene Gesamtkonzentration an (i) Platin, Rhodium, Nickel und Iridium in metallischer Form und in Form von in Verbindungen dieser Metalle enthaltenem Platin, Rhodium, Nickel und Iridium im ersten heterogenen Katalysator in den vorstehend genannten bevorzugten Bereichen.

Für die erfindungsgemäße Umsetzung in Gegenwart des ersten heterogenen Katalysators ist molekularer Wasserstoff in einer mindestens äquimolaren Menge zuzudosieren. Als günstiger hat sich jedoch erwiesen, Wasserstoff in einem Überschuss zuzudosieren. Daher beträgt bei der Umsetzung in Gegenwart des ersten heterogenen Katalysators das molare Verhältnis zwischen Wasserstoff und der Gesamtmenge an Furfurylalkohol und Furfural 1:1 oder mehr und liegt bevorzugt im Bereich von 4:1 bis 100:1, besonders bevorzugt im Bereich von 5:1 bis 20:1.

Bei der Umsetzung in Gegenwart des ersten heterogenen Katalysators liegt der Wasserstoffpartialdruck im Rahmen der vorliegenden Erfindung im Bereich von 0,1 bis 0,8 MPa (1 bar bis 8 bar), bevorzugt im Bereich von 0,1 bis 0,4 MPa(1 bar bis 4 bar).

Die Umsetzung in Gegenwart des ersten heterogenen Katalysators erfolgt in einer Flüssigphase oder in einer Gasphase.

Wird die Umsetzung in Gegenwart des ersten heterogenen Katalysators in einer Gasphase durchgeführt, so erfolgt dies bevorzugt bei einer Temperatur im Bereich von 100 °C bis 250 °C, weiter bevorzugt im Bereich von 150 °C bis 240 °C, besonders bevorzugt im Bereich von 170 °C bis 230 °C. Bevorzugt liegen die Temperatur und der Wasserstoffpartialdruck in den vorstehend genannten bevorzugten Bereichen.

Bevorzugt erfolgt dabei die Umsetzung in Gegenwart des ersten heterogenen Katalysators in einem Reaktor, der kontinuierlich von einem Gasstrom durchströmt wird. Dieser Gasstrom umfasst beim Eintritt in den Reaktor das Edukt (wie oben beschrieben) sowie Wasserstoff und optional ein Inertgas, wobei
- die Strömungsrate dieses Gasstroms bezogen auf das Volumen des ersten heterogenen Katalysators (Gas hourly space velocity GHSV) 500 h⁻¹ bis 5000 h⁻¹, bevorzugt 900 h⁻¹ bis 3600 h⁻¹ beträgt
   und/oder
- die Gesamtkonzentration an Furfurylalkohol und Furfural in dem in den Reaktor eintretenden Gasstrom 1 Mol-% bis 15 Mol-%, bevorzugt 3 Mol-% bis 10 Mol-% beträgt.

Bevorzugt ist sowohl die Bedingung hinsichtlich der Strömungsgeschwindigkeit als auch die Bedingung hinsichtlich der Gesamtkonzentration an Furfurylalkohol und Furfural in dem in den Reaktor eintretenden Gasstrom erfüllt.

Als Apparat für die Umsetzung in Gegenwart des ersten heterogenen Katalysators in einer Gasphase wird bevorzugt ein Rohrreaktor eingesetzt, der mit einem ersten heterogenen Katalysator wie oben definiert bestückt ist. Der Zustrom, der dem Rohrreaktor über geeignete Dosiervorrichtungen zugeleitet wird, besteht aus einem Edukt umfassend eine oder beide Verbindungen aus der Gruppe bestehend aus Furfurylalkohol und Furfural und der notwendigen Menge Wasserstoff (bevorzugt im oben angegebenen molaren Verhältnis zwischen Wasserstoff und der Gesamtmenge an Furfurylalkohol und Furfural) und gegebenenfalls einem Inertgas. Furfurylalkohol und/oder Furfural werden mittels eines Sättigers, der das flüssige Furfurylalkohol und/oder Furfural auf eine Temperatur im Bereich von 80 und 120 °C, besonders bevorzugt 90 bis 110 °C, erwärmt und von Wasserstoff oder Wasserstoff und einem Inertgas durchströmt wird, in die Gasphase überführt. Alternativ wird das flüssige Ausgangsmaterial über eine Dosierpumpe oder ähnliche Vorrichtungen und einen Verdampfer eingespeist.

Wird die Umsetzung in Gegenwart des ersten heterogenen Katalysators in einer flüssigen Phase durchgeführt, so erfolgt dies bevorzugt bei einer Temperatur im Bereich von -20 °C bis +100 °C, bevorzugt von -20 °C bis + 50 °C, weiter bevorzugt im Bereich von -5 bis + 50 °C, noch weiter bevorzugt im Bereich von -5 °C bis +30 °C, besonders bevorzugt im Bereich von 0 °C bis +30 °C, ganz besonders bevorzugt im Bereich von 0 °C bis +10 °C. Bevorzugt liegen die Temperatur und der Wasserstoffpartialdruck in den vorstehend genannten bevorzugten Bereichen.

Die Reaktionszeit für die Umsetzung in Gegenwart des ersten heterogenen Katalysators wie oben definiert liegt vorzugsweise im Bereich von 1 bis 20 Stunden, bevorzugt im Bereich von 2 bis 12 Stunden, weiter bevorzugt im Bereich von 3 bis 8 Stunden.

Erfindungsgemäß wird die Umsetzung von Furfurylalkohol mit Wasserstoff vorzugsweise in einem oder mehreren, vorzugsweise organischen, Verdünnungsmitteln mit einem pKₛ-Wert bei 25 °C von größer oder gleich 6 durchgeführt, vorzugsweise mit einem pKₛ-Wert bei 25 °C von größer oder gleich 8, bevorzugt mit einem pKₛ-Wert bei 25° C von größer oder gleich 10, besonders bevorzugt mit einem pKₛ-Wert bei 25 °C von größer oder gleich 12.

Weiter bevorzugt sind organische Verdünnungsmittel mit einem pKₛ-Wert bei 25 °C im Bereich von 12 bis 25, insbesondere bevorzugt im Bereich von 13 bis 20, am meisten bevorzugt im Bereich von 14 bis 18.

Der pKₛ-Wert (auch pKₐ-Wert) entspricht dem negativen dekadischen Logarithmus der Säurekonstante Kₛ. Essigsäure hat eine pKₛ-Wert von 4,75.

Die erfindungsgemäß einzusetzenden Verdünnungsmittel sind dabei vorzugsweise unter den Reaktionsbedingungen inert, d.h. dass die Verdünnungsmittel unter den herrschenden Hydrierungs- bzw. Hydrogenolysebedingungen vorzugsweise nicht selbst reagieren, insbesondere selbst nicht reduziert werden.

Bevorzugt einzusetzende Verdünnungsmittel sind oder umfassen einen oder mehrere Alkohole mit 1 bis 4 Kohlenstoffatomen, vorzugsweise ausgewählt aus der Gruppe bestehend aus Methanol, Ethanol, n-Propanol, Isopropanol und deren Mischungen.

Es können auch unpolare und/oder aprotische, inerte Lösungsmittel wie Dibutylether, Methyl-tert.-butylether (MTBE), Cyclohexan, n-Octan, Isooctan oder Decalin verwendet werden, optional in Kombination mit einem oder mehreren der vorstehend als bevorzugt gekennzeichneten Alkohole mit 1 bis 4 Kohlenstoffatomen.

In einer bevorzugten Ausgestaltung kann zusätzlich zu einem Verdünnungsmittel, vorzugsweise zusätzlich zu einem der vorstehend als bevorzugt gekennzeichneten Verdünnungsmittel, eine anorganische Säure, vorzugsweise mit einem pKₛ-Wert bei 25 °C von kleiner als 3, bevorzugt mit einem pKₛ-Wert bei 25 °C von kleiner als 0, eingesetzt werden. Eine bevorzugte anorganische Säure ist dabei Schwefelsäure, da diese sich als besonders günstig für den Verlauf der Hydrogenolyse in dem erfindungsgemäßen Verfahren gezeigt hat.

In einer besonders bevorzugten Ausgestaltung erfolgt die Umsetzung von Furfurylalkohol in einem Verdünnungsmittel umfassend oder bestehend aus einem oder mehreren Alkoholen ausgewählt aus der Gruppe bestehend aus Methanol, Ethanol, n-Propanol, Isopropanol und deren Mischungen, und Schwefelsäure. Besonders bevorzugt ist dabei die Kombination von Ethanol und Schwefelsäure.

Sofern eine anorganische Säure eingesetzt wird, vorzugsweise an Schwefelsäure, liegt die Gesamtmenge vorzugsweise im Bereich von 0,0001 bis 1 Gew.-%, bevorzugt im Bereich von 0,001 bis 0,5 Gew.-%, besonders bevorzugt im Bereich von 0,01 bis 0,1 Gew.-%, bezogen auf die Gesamtmasse des oder der Verdünnungsmittel, wobei die Verdünnungsmittel wiederum vorzugsweise ausgewählt sind aus der Gruppe bestehend aus Methanol, Ethanol, n-Propanol, Isopropanol und deren Mischungen.

Die Gesamtmenge des oder der Verdünnungsmittel, vorzugsweise mit einem oben als bevorzugt bzw. besonders bevorzugt gekennzeichneten pKₛ-Wert bei 25 °C, bevorzugt ausgewählt aus der Gruppe der oben als bevorzugt gekennzeichneten Verdünnungsmittel, liegt vorzugsweise im Bereich von 25 bis 1000 Gew.-%, bevorzugt im Bereich von 50 bis 500 Gew.-%, weiter bevorzugt im Bereich von 100 bis 300 Gew.-%, bezogen auf die insgesamt eingesetzte Menge an Furfurylalkohol.

Ein erfindungsgemäß bevorzugt einzusetzender erster heterogener Katalysator ist ein heterogener Platin-Katalysator umfassend (i) Platin und/oder eine oder mehrere Platinverbindungen (insbesondere Platin(IV)-Verbindungen) und (ii) ein oder mehrere Trägermaterialien. Ein erfindungsgemäß besonders bevorzugt einzusetzender heterogener Platin-Katalysator umfasst (i) Platin und/oder eine oder mehrere Platin(IV)-Verbindungen und (ii) ein oder mehrere Trägermaterialien.

Bevorzugte Platin(IV)-Verbindungen sind H₂PtCl₆ und deren Salze, dabei bevorzugt (NH₄)₂PtCl₆, sowie Platindioxid (dabei wiederum bevorzugt sind PtO₂ und PtO₂-Hydrat). Eine besonders bevorzugte Platin(IV)-Verbindung ist Platindioxid PtO₂.

Bevorzugt ist elementares, d.h. metallisches, Platin, welches Eisen, Vanadium und/oder Ruthenium dotiert sein kann.

Das Trägermaterial ist vorzugsweise bei 25 °C und 1013 mbar fest, vorzugsweise auch unter Hydrierbedingungen. Das Trägermaterial ist dabei vorzugsweise ausgewählt aus der Gruppe bestehend aus Aktivkohle, Kieselsäure, Siliciumdioxid und/oder Aluminiumoxid.

Besonders bevorzugte Trägermaterialien sind Aluminiumoxid und Aktivkohle, da erfindungsgemäß einzusetzende Platin-Katalysatoren umfassend Aluminiumoxid und/oder Aktivkohle die besten Ausbeuten und die besten Selektivitäten an 1,2-Pentandiol ergaben.

Bevorzugt umfasst daher das Trägermaterial Aktivkohle und/oder Aluminiumoxid, besteht aus Aluminiumoxid oder besteht aus Aktivkohle.

Als besonders gutes Trägermaterial hat sich gamma- Aluminiumoxid herausgestellt.

Besonders gute Ergebnisse in einem erfindungsgemäßen Verfahren wurden mit Platindioxid auf Aluminiumoxid, bzw. Platin auf Aktivkohle oder Aluminiumoxid erzielt, die besten Ergebnisse mit Platindioxid auf Aluminiumoxid, insbesondere mit Platindioxid auf gamma-Aluminiumoxid.

Die erfindungsgemäß einzusetzenden heterogenen Platin-Katalysatoren sind an sich bekannt und können beispielsweise gemäß Anal. Chem. 1956, 28(3), 362-365 bzw. Thermochimica Acta 1977, 20(3), 297-308 oder Proceedings of the 13th International Conference On X-Ray Absorption Fine Structure (XAFS13), Stanford, California, 2006, edited by B. Hedman and P. Pianetta, eConf C060709 (2006) (der Volltext ist verfügbar unter http://www.slac.stanford.edu/econf/C060709/papers/207_WEPO17.PDF) oder in Analogie dazu erhalten werden.

Die Menge des heterogenen Platin-Katalysators liegt vorzugsweise im Bereich von 0,1 bis 20 Gew.-%, bevorzugt im Bereich von 0,25 bis 15 Gew.-%, weiter bevorzugt im Bereich von 0,5 bis 12 Gew.-%, bezogen auf die eingesetzte Menge an Furfurylalkohol.

Der Anteil des Bestandteils (i) des heterogenen Platin-Katalysators, d.h. der Gesamtgehalt an Platin und/oder Platinverbindungen, liegt vorzugsweise im Bereich von 0,5 bis 50 Gew.-%, bevorzugt im Bereich von 0,5 bis 20 Gew.-%, weiter bevorzugt im Bereich von 0,5 bis 10 Gew.-%, bezogen auf die Gesamtmenge des heterogenen Platin-Katalysators.

Besonders bevorzugte Platin-Katalysatoren in einem erfindungsgemäßen Verfahren sind Platindioxid auf Aluminiumoxid, vorzugsweise Platindioxid auf gamma-Aluminiumoxid, und/oder Platin auf Aktivkohle, wobei der Gesamtgehalt an Platindioxid und/oder Platin im Bereich von 0,5 bis 10 Gew.-% liegt, bezogen auf die Gesamtmenge des eingesetzten heterogenen Platin-Katalysators.

Vorzugsweise ist ein erfindungsgemäß als erster heterogener Katalysator einzusetzender Platin-Katalysator (wie oben definiert) frei von ReO₂ und frei von Cr₂O₃, bevorzugt frei von Rhenium und frei von Chrom. Vorzugsweise ist ein erfindungsgemäß als erster heterogener Katalysator einzusetzender Platin-Katalysator (wie oben definiert) frei von CeO₂, bevorzugt frei von Cer-oxiden, weiter bevorzugt frei von Cer, Rhenium und Chrom. Vorzugsweise ist ein erfindungsgemäß als erster heterogener Katalysator einzusetzender Platin-Katalysator (wie oben definiert)frei von Oxiden der Elemente Re, Mo, Mn, Co, Ti, W, Cr, Fe, V und Ta.

In der bevorzugten Variante des erfindungsgemäßen Verfahrens mit einem heterogenen Platinkatalysator wie oben definiert liegt das Massenverhältnis von Furfurylalkohol zu der Gesamtmenge an Bestandteil (i) des heterogenen Platin-Katalysators vorzugsweise im Bereich von 2000 : 1 bis 10 : 1, bevorzugt im Bereich von 1000 : 1 bis 25 : 1, weiter bevorzugt im Bereich von 500 : 1 bis 50 : 1, am meisten bevorzugt im Bereich von 300 : 1 bis 100 : 1.

Vorzugsweise wird die Hydrogenolyse von Furfurylalkohol bei dem erfindungsgemäßen Verfahren bei einer Temperatur im Bereich von -20 °C bis +50 °C durchgeführt, bevorzugt im Bereich von -5 bis +30 °C, weiter bevorzugt im Bereich von 0 bis +10 °C.

Der Wasserstoffdruck bei der bevorzugten Variante des erfindungsgemäßen Verfahrens mit einem heterogenen Platinkatalysator wie oben definiert liegt im Bereich von 0,1 bis 0,8 MPa (1 bis 8 bar), weiter bevorzugt im Bereich von 0,1 bis 0,4 MPa (1 bis 4 bar).

Die Reaktionszeit bei der bevorzugten Variante des erfindungsgemäßen Verfahrens mit einem heterogenen Platinkatalysator wie oben definiert liegt vorzugsweise im Bereich von 1 bis 20 Stunden, bevorzugt im Bereich von 2 bis 12 Stunden, weiter bevorzugt im Bereich von 3 bis 8 Stunden.

Das erfindungsgemäße Verfahren führt zu einem Produktgemisch, in dem 1,2-Pentandiol regelmäßig das Hauptprodukt darstellt.

Das nachfolgende Reaktionsschema veranschaulicht die Umsetzung von Furfurylalkohol in einem erfindungsgemäßen Verfahren zu dem gewünschten Hauptprodukt 1,2-Pentandiol und den dabei regelmäßig erhaltenen Nebenprodukten.

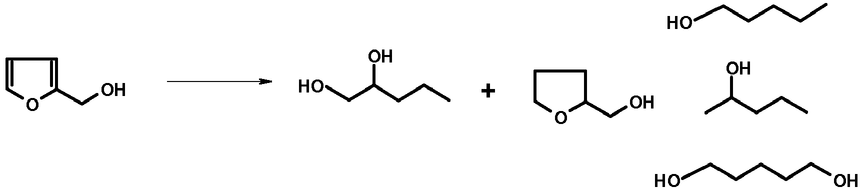

Neben den im Reaktionsschema dargestellten Nebenprodukten, die dank der guter Ausbeute und/oder guten Selektivität des erfindungsgemäßen Verfahrens nur in geringen Anteilen gebildet werden, entsteht neben dem Zielprodukt 1,2-Pentandiol insbesondere 1-Hydroxy-2-pentanon. Dieses wird in einer vorteilhaften Weiterentwicklung des erfindungsgemäßen Verfahrens (siehe unten) in einem weiteren Umsetzungsschritt ebenfalls zu dem Zielprodukt 1,2-Pentandiol umgesetzt.

Vorzugsweise erfolgt die Reaktionsführung in der Weise, dass zumindest 80 Gew.-%, bevorzugt zumindest 90 Gew.-%, weiter bevorzugt zumindest 95 Gew.-% des eingesetzten Furfurylalkohols umgesetzt werden.

Vorzugsweise erfolgt die Reaktionsführung in der Weise, dass das nach beendeter Umsetzung vorliegende Reaktionsgemisch zumindest 40 Gew.-% an 1,2-Pentandiol enthält, bevorzugt zumindest 50 Gew.-%, weiter bevorzugt zumindest 60 Gew.-%, besonders bevorzugt zumindest 70 Gew.-%, bezogen auf die Gesamtmenge der entstandenen Produkte.

Zur Klarstellung: die angegebenen Gewichtsanteile an 1,2-Pentandiol in dem Reaktionsgemisch beziehen sich dabei nur auf die Gesamtmenge der aus Furfurylalkohol entstandenen Produkte. Dementsprechend nicht zu berücksichtigen sind für die Ermittlung der oben angegebenen Gewichtsanteile an 1,2-Pentandiol eventuell in dem Reaktionsgemisch nach Beendigung der Umsetzung noch vorhandene Mengen an nicht umgesetzten Furfurylalkohol sowie die Mengen an Platin-Katalysator(en) und Verdünnungsmittel(n).

Die oben angegebenen Gewichtsanteile an 1,2-Pentandiol in dem Reaktionsgemisch beziehen sich insbesondere auf die Gesamtmenge an 1-Pentandiol, 2-Pentandiol, Tetrahydrofurfurol, 1,2-Pentandiol und 1,5-Pentandiol in dem Reaktionsgemisch nach Beendigung der Umsetzung von Furfurylalkohol.

Das erfindungsgemäße Verfahren kann in der Flüssigphase oder in der Gasphase durchgeführt werden.

Die Reaktionsdurchführung kann dabei kontinuierlich, semi-kontinuierlich oder diskontinuierlich erfolgen.

Bevorzugt ist die Reaktionsdurchführung in einem Batch-Verfahren, vorzugsweise in einem Reaktionskessel, wobei der Furfurylalkohol und vorzugsweise das optional eingesetzte Verdünnungsmittel in flüssiger Phase vorliegen und mit dem erfindungsgemäß einzusetzenden Katalysator gemischt werden.

Bevorzugt ist die Reaktionsdurchführung in einem Reaktionsrohr, wobei der erfindungsgemäß einzusetzende Katalysator als Festbett ausgestaltet ist und der Furfurylalkohol und vorzugsweise das optional eingesetzte Verdünnungsmittel in flüssiger Phase vorliegen und mit dem Katalysator-Festbett in Kontakt gebracht werden.

Das erfindungsgemäße Verfahren zur Herstellung von 1,2-Pentandiol durch Umsetzung von Furfurylalkohol in Gegenwart eines heterogenen Platin-Katalysators ist vorzugsweise gekennzeichnet durch folgende Schritte:
(a) Bereitstellen eines Edukts umfassend eine oder beide Verbindungen aus der Gruppe bestehend aus Furfurylalkohol und Furfural;
(b) Bereitstellen eines ersten heterogenen Katalysators wie oben definiert;
(c) optional Bereitstellen eines oder mehrerer Verdünnungsmittel;
(d) Herstellen einer Mischung umfassend die in den Schritten (a) und (b) sowie optional (c) bereitgestellten Komponenten und Kontaktieren dieser Mischung mit Wasserstoff oder
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator;
(e) in der in Schritt (d) bzw. (d') hergestellten Mischung Umsetzen des Edukts mit Wasserstoff in Gegenwart des ersten heterogenen Katalysators unter Bildung eines Gemisches enthaltend 1,2-Pentandiol und gegebenenfalls eine oder mehrere Verbindungen aus der Gruppe bestehend aus 1-Hydroxy-2-pentanon, Furfurylalkohol und Furfural;
(f) optional destillative Abtrennung des 1,2-Pentandiol.

Dabei sind die oben als bevorzugt bzw. besonders bevorzugt angegebenen Verdünnungsmittel hier entsprechend bevorzugt bzw. besonders bevorzugt.

Dabei sind die oben als bevorzugt bzw. besonders bevorzugt angegebenen ersten heterogenen Katalysatoren hier entsprechend bevorzugt bzw. besonders bevorzugt.

Dabei werden vorzugsweise eine, mehrere oder sämtliche der oben als bevorzugt bzw. besonders bevorzugt angegebenen Reaktionsbedingungen eingestellt.

In welcher Reihenfolge in Schritt (d) die in den Schritten (a) bis (c) bereitgestellten Komponenten miteinander gemischt werden, ist dabei unerheblich.

In einer besonders bevorzugten Variante umfasst das erfindungsgemäße Verfahren die Schritte:
(a) Bereitstellen von Furfurylalkohol,
(b) Bereitstellen mindestens eines ersten heterogenen Katalysators wie oben definiert, vorzugsweise eines heterogenen Platin-Katalysators, welcher (i) Platin und/oder eine Platinverbindung und (ii) ein oder mehrere Trägermaterialien umfasst,
(c) optional Bereitstellen eines oder mehrerer Verdünnungsmittel, vorzugsweise mit einem pKₛ-Wert bei 25 °C von größer oder gleich 6,
(d) Herstellung einer Mischung umfassend die in den Schritten (a) und (b) sowie vorzugsweise (c) bereitgestellten Komponenten,
(e) Kontaktieren der in Schritt (d) hergestellten Mischung mit Wasserstoff.

In einer alternativen bevorzugten Variante umfasst das erfindungsgemäße Verfahren die Schritte:
(a) Bereitstellen eines Edukts umfassend eine oder beide Verbindungen aus der Gruppe bestehend aus Furfurylalkohol und Furfural;
(b) Bereitstellen eines ersten heterogenen Katalysators wie oben definiert;
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator;
(e) in der in Schritt (d') hergestellten Mischung Umsetzen des Edukts mit Wasserstoff in Gegenwart des ersten heterogenen Katalysators unter Bildung eines Gemisches enthaltend 1,2-Pentandiol und gegebenenfalls eine oder mehrere Verbindungen aus der Gruppe bestehend aus 1-Hydroxy-2-pentanon, Furfurylalkohol und Furfural.

Mit dem vorstehend beschriebenen erfindungsgemäßen Verfahren ist es möglich, durch Umsetzung eines Edukts umfassend eine oder beide Verbindungen aus der Gruppe bestehend aus Furfurylalkohol und Furfural Ausbeuten an 1,2-Pentandiol erzielt werden, die den bekannten Stand der Technik übertreffen. Ein weiteres vorteilhaftes Merkmal des erfindungsgemäßen Verfahrens ist die Bildung des Nebenprodukts 1-Hydroxy-2-pentanon mit hoher Ausbeute und Selektivität. Andere Nebenprodukte, die bei aus dem Stand der Technik bekannten Verfahren typischerweise entstehen, werden bei dem erfindungsgemäßen Verfahren hingegen nur noch in sehr geringen Mengen gebildet. Insbesondere sind in dem durch das erfindungsgemäße Verfahren erhältlichen Gemisch kaum cyclische Verbindungen nachweisbar.

In der Regel erreicht die Summe der Selektivitäten für 1-Hydroxy-2-pentanon und 1,2-Pentandiol über 80 %, auch bei fast vollständigem Umsatz. Die Verbindung 1-Hydroxy-2-Pentanon wurde in Untersuchungen zu aus dem Stand der Technik bekannten Verfahren zur Herstellung von 1,2-Pentandiol nicht als Nebenprodukt nachgewiesen. Die Bildung von 1-Hydroxy-2-pentanon ist gegenüber anderen Nebenprodukten von Vorteil, da 1-Hydroxy-2-pentanon unter geeigneten Bedingungen in Gegenwart eines zweiten heterogenen Katalysators leicht und sehr selektiv zu 1,2-Pentandiol hydriert werden kann. Dies ist Gegenstand einer vorteilhaften Weiterentwicklung des erfindungsgemäßen Verfahrens, die nachfolgend beschrieben wird.

In einer vorteilhaften Weiterentwicklung umfasst das erfindungsgemäße Verfahren, insbesondere in den oben beschriebenen bevorzugten Varianten, daher den weiteren Schritt
- Umsetzung von Verbindungen aus der Gruppe bestehend aus 1-Hydroxy-2-pentanon, Furfurylalkohol und Furfural aus dem in der oben beschriebenen Umsetzung in Gegenwart des ersten heterogenen Katalysators gebildeten Gemisch mit Wasserstoff in Gegenwart eines zweiten heterogenen Katalysators unter Bildung von 1,2-Pentandiol,
   wobei der zweite heterogene Katalysator
   (i') ein oder mehrere Metalle aus der Gruppe bestehend aus Platin, Rhodium, Nickel und Iridium in metallischer Form und/oder eine oder mehrere Verbindungen von Metallen aus der Gruppe bestehend aus Platin, Rhodium, Nickel und Iridium,
      sowie
   (ii') ein oder mehrere Trägermaterialien
      umfasst.

Mit dieser vorteilhaften Weiterentwicklung des erfindungsgemäßen Verfahrens lässt sich die Gesamtselektivität für 1,2-Pentandiol auf über 80 % steigern. Eine Abtrennung des 1,2-Pentandiols vor der Umsetzung in Gegenwart des zweiten heterogenen Katalysators ist nicht notwendig.

Bevorzugt liegt im zweiten heterogenen Katalysator die Gesamtkonzentration an Platin, Rhodium, Nickel und Iridium in metallischer Form und in Form von in Verbindungen dieser Metalle enthaltenem Platin, Rhodium, Nickel und Iridium im Bereich von 0,1 Gew.-% bis 20 Gew.-%, bevorzugt von 0,5 Gew.-% bis 10 Gew.-%, bezogen auf das Gesamtgewicht aller Bestandteile des zweiten heterogenen Katalysators.

In einer bevorzugten Variante dieser vorteilhaften Weiterentwicklung des erfindungsgemäßen Verfahrens haben der erste und der zweite heterogene Katalysator die gleiche Zusammensetzung.

Bevorzugt ist das Trägermaterial (ii) des ersten heterogenen Katalysators und/oder das Trägermaterial (ii') des zweiten heterogenen Katalysators bei 25 °C und 0.1013 MPa (1013 mbar), vorzugsweise auch bei 230 °C und 0.1013 MPa (1013 mbar), fest, wobei vorzugsweise das Trägermaterial (ii) und/oder das Trägermaterial (ii') ausgewählt ist aus der Gruppe bestehend aus Aktivkohle, Kieselsäure, Siliciumdioxid, Siliciumcarbid, Aluminiumoxid, Zirconiumdioxid, Titandioxid, Niobtrioxid, Ceriumdioxid und deren Gemischen.

Die Umsetzung in Gegenwart des zweiten heterogenen erfolgt in einer Flüssigphase oder in einer Gasphase.

Wird die Umsetzung in Gegenwart des zweiten heterogenen Katalysators in einer Gasphase durchgeführt, so liegt dabei die Temperatur bevorzugt im Bereich von 25 °C bis 240 °C, besonders bevorzugt im Bereich von 100 °C bis 130 °C. Bevorzugt liegen die Temperatur und der Wasserstoffpartialdruck in den hier genannten bevorzugten Bereichen.

Bevorzugt erfolgt dabei die Umsetzung in Gegenwart des zweiten heterogenen Katalysators in einem Reaktor, der kontinuierlich von einem Gasstrom durchströmt wird. Dieser Gasstrom umfasst beim Eintritt in den Reaktor das bei der Umsetzung in Gegenwart des ersten heterogenen Katalysators (wie oben beschrieben) gebildete Gemisch sowie Wasserstoff und optional ein Inertgas, wobei
- die Strömungsrate dieses Gasstroms bezogen auf das Volumen des zweiten heterogenen Katalysators (Gas hourly space velocity GHSV) 500 h⁻¹ bis 5000 h⁻¹, bevorzugt 900 h⁻¹ bis 3600 h⁻¹ beträgt
   und/oder
- die Gesamtkonzentration an 1-Hydroxy-2-pentanon, Furfurylalkohol und Furfural in dem in den Reaktor eintretenden Gasstrom 1 Mol-% bis 15 Mol-%, bevorzugt 3 Mol-% bis 10 Mol-% beträgt.

Bevorzugt ist sowohl die Bedingung hinsichtlich der Strömungsgeschwindigkeit als auch die Bedingung hinsichtlich der Gesamtkonzentration an 1-Hydroxy-2-pentanon, Furfurylalkohol und Furfural in dem in den Reaktor eintretenden Gasstrom erfüllt.

In einer bevorzugten Variante des erfindungsgemäßen Verfahrens werden sowohl die Umsetzung in Gegenwart des ersten heterogenen Katalysators als auch die Umsetzung in Gegenwart des zweiten heterogenen Katalysators in der Gasphase ausgeführt. Diese Variante hat den Vorteil, dass keine Verdünnungsmittel (Lösungsmittel) benötigt werden, was einen erheblichen ökonomischen und ökologischen Vorteil darstellt. Bevorzugt wird für diese Variante des erfindungsgemäßen Verfahrens eine Apparatur eingesetzt umfassend ein erstes Reaktionsrohr und ein zweites Reaktionsrohr, das dem ersten Reaktionsrohr nachgeschaltet ist. Im ersten Reaktionsrohr ist der erste heterogene Katalysator angeordnet, im zweiten Reaktionsrohr der zweite heterogene Katalysator. Dabei ist es möglich, im ersten Reaktionsrohr eine andere Temperatur einzustellen als im zweiten Reaktionsrohr.

Wird die Umsetzung in Gegenwart des zweiten heterogenen Katalysators in einer Flüssigphase durchgeführt, so liegt die Temperatur im Bereich von -20 °C bis +150 °C, bevorzugt von -20 °C bis + 50 °C, weiter bevorzugt im Bereich von -5 bis + 50 °C, noch weiter bevorzugt im Bereich von -5 °C bis +30 °C, besonders bevorzugt im Bereich von 0 °C bis +30 °C, ganz besonders bevorzugt im Bereich von 0 °C bis +10 °C. Bevorzugt liegen die Temperatur und der Wasserstoffpartialdruck in den hier genannten bevorzugten Bereichen.

Bevorzugt umfasst dabei die flüssige Phase ein oder mehrere, vorzugsweise polare, Verdünnungsmittel, wobei das oder die Verdünnungsmittel bevorzugt ausgewählt sind aus der Gruppe bestehend aus Wasser, Alkoholen mit 1 bis 4 Kohlenstoffatomen, Ethern ausgewählt aus der Gruppe bestehend aus aliphatischen Ethern, oligomeren terminal hydroxyfunktionalisierten Ethern und cyclischen Ethern, und deren Mischungen. Die erfindungsgemäß einzusetzenden Verdünnungsmittel sind dabei vorzugsweise unter den Hydrierungsbedingungen inert, d.h. dass die Verdünnungsmittel unter den herrschenden Hydrierungsbedingungen vorzugsweise nicht selbst reagieren, insbesondere selbst nicht reduziert werden.

Bevorzugt als Verdünnungsmittel einzusetzende Alkohole mit 1 bis 4 Kohlenstoffatomen sind ausgewählt aus der Gruppe bestehend aus Methanol, Ethanol, n-Propanol, Isopropanol, n-Butanol, iso-Butanol, tert.-Butanol. Bevorzugt als Verdünnungsmittel einzusetzende Ether sind ausgewählt aus der Gruppe bestehend aus aliphatischen Ethern wie Methyl-tert.-butylether, oligomeren terminal hydroxyfunktionalisierten Ethern wie Diethylenglykol und Triethylenglycol, cyclischen Ethern wie Tetrahydrofuran und Dioxan oder deren Mischungen.

Als Apparat wird für die Umsetzung in Gegenwart des zweiten heterogenen Katalysators bevorzugt ein Rührkesselreaktor eingesetzt. Die Reaktionszeit für die Umsetzung in Gegenwart des zweiten heterogenen Katalysators im Rührkesselreaktor liegt vorzugsweise im Bereich von 0,25 bis 20 Stunden, bevorzugt im Bereich von 2 bis 12 Stunden, weiter bevorzugt im Bereich von 3 bis 8 Stunden.

In einer besonders bevorzugten Variante des erfindungsgemäßen Verfahrens erfolgt die Umsetzung eines Edukts umfassend eine oder beide Verbindungen aus der Gruppe bestehend aus Furfurylalkohol und Furfural in Gegenwart eines ersten heterogenen Katalysators in einer Gasphase und die Umsetzung von Verbindungen aus der Gruppe bestehend aus 1-Hydroxy-2-pentanon, Furfurylalkohol und Furfural aus dem in Gegenwart des ersten heterogenen Katalysators gebildeten Gemisch in Gegenwart eines zweiten heterogenen Katalysators mit Wasserstoff unter Bildung in einer flüssigen Phase. Dazu werden die organischen Komponenten des in Gegenwart des ersten heterogenen Katalysators gebildeten Gemisches in einer geeigneten Vorrichtung auskondensiert (d.h. in die flüssige Phase überführt). Das erhaltene Kondensat wird dann in einen Reaktor, vorzugsweise einen Rührkesselreaktor, überführt zur Durchführung der Umsetzung in Gegenwart des zweiten heterogenen Katalysators. Vorzugsweise werden bei dieser Verfahrensweise für die Umsetzung in Gegenwart des ersten heterogenen Katalysators solche Reaktionsbedingungen eingestellt, die zu einem nahezu vollständigen Umsatz der Ausgangsverbindung Furfurylalkohol oder Furfural führen.

Dem gebildeten Kondensat werden vorzugsweise ein oder mehrere Verdünnungsmittel zugesetzt, wobei vorzugsweise die Gesamtmenge der dem Kondensat zugesetzten Verdünnungsmittel im Bereich von 25 bis 1000 Gew.-% liegt, bevorzugt im Bereich von 50 bis 500 Gew.-%, weiter bevorzugt im Bereich von 100 bis 300 Gew.-%, bezogen auf die Masse des Kondensats.

Die Gesamtkonzentration an (i) Platin, Rhodium, Nickel und Iridium in metallischer Form und in Form von in Verbindungen dieser Metalle enthaltenem Platin, Rhodium, Nickel und Iridium im zweiten heterogenen Katalysator liegt dabei bevorzugt im Bereich von 0,01 bis 10 Mol.-%, bezogen auf die im Kondensat enthaltene Stoffmenge an 1-Hydroxy -2-Pentanon.

Das erfindungsgemäße Verfahren gemäß der vorstehend beschriebenen vorteilhaften Weiterentwicklung ist vorzugsweise gekennzeichnet durch folgende Schritte:
(a) Bereitstellen eines Edukts umfassend eine oder beide Verbindungen aus der Gruppe bestehend aus Furfurylalkohol und Furfural,
(b) Bereitstellen eines ersten heterogenen Katalysators wie oben definiert,
(c) optional Bereitstellen eines oder mehrerer Verdünnungsmittel,
(d) Herstellen einer Mischung umfassend die in den Schritten (a) und (b) sowie optional (c) bereitgestellten Komponenten und Kontaktieren dieser Mischung mit Wasserstoff oder
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator,
(e) in der in Schritt (d) bzw. (d') hergestellten Mischung Umsetzen des Edukts mit Wasserstoff in Gegenwart des ersten heterogenen Katalysators unter Bildung eines Gemisches enthaltend 1,2-Pentandiol und eine oder mehrere Verbindungen aus der Gruppe bestehend aus 1-Hydroxy-2-pentanon, Furfurylalkohol und Furfural,
(f) Bereitstellen eines zweiten heterogenen Katalysators wie oben definiert,
(g) optional Bereitstellen eines oder mehrerer Verdünnungsmittel,
(h) gegebenenfalls Auskondensieren organischer Komponenten des in Schritt (e) gebildeten Gemischs und Herstellen einer Mischung umfassend das in Schritt (e) gebildeten Gemisch bzw. daraus auskondensierte organische Komponenten und die in den Schritten (f) und optional (g) bereitgestellten Komponenten und Kontaktieren dieser Mischung mit Wasserstoff
   oder
(h') Kontaktieren des in Schritt (e) gebildeten Gemischs in Gegenwart von Wasserstoff und optional eines Inertgases mit dem gemäß Schritt (f) bereitgestellten zweiten heterogenen Katalysator,
(i) Umsetzen von Verbindungen aus der Gruppe bestehend aus 1-Hydroxy-2-pentanon, Furfurylalkohol und Furfural aus dem in Schritt (e) gebildeten Gemisch in Gegenwart des zweiten heterogenen Katalysators mit Wasserstoff unter Bildung von 1,2-Pentandiol,
(j) optional destillatives Abtrennen des 1,2-Pentandiol.

Dabei sind die oben als bevorzugt bzw. besonders bevorzugt angegebenen Verdünnungsmittel hier entsprechend bevorzugt bzw. besonders bevorzugt.

Dabei sind die oben als bevorzugt bzw. besonders bevorzugt angegebenen heterogenen Katalysatoren hier entsprechend bevorzugt bzw. besonders bevorzugt.

Dabei werden vorzugsweise eine, mehrere oder sämtliche der oben als bevorzugt bzw. besonders bevorzugt angegebenen Reaktionsbedingungen eingestellt.

In welcher Reihenfolge in Schritt (d) die in den Schritten (a) bis (c) bereitgestellten Komponenten miteinander gemischt werden, ist dabei unerheblich.

Nicht umgesetzter Wasserstoff kann in allen erfindungsgemäßen Verfahrensvarianten beispielsweise in einem Kondensator sehr leicht von den organischen Bestandteilen des Reaktionsmischung abgetrennt und der Umsetzung in Gegenwart des ersten heterogenen Katalysators wieder zugeführt werden.

In einer ersten bevorzugten Variante umfasst das erfindungsgemäße Verfahren die Schritte:
(a) Bereitstellen eines Edukts umfassend eine oder beide Verbindungen aus der Gruppe bestehend aus Furfurylalkohol und Furfural,
(b) Bereitstellen eines ersten heterogenen Katalysators wie oben definiert,
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator,
(e) in der in Schritt (d') hergestellten Mischung Umsetzen des Edukts mit Wasserstoff in Gegenwart des ersten heterogenen Katalysators unter Bildung eines Gemisches enthaltend 1,2-Pentandiol und eine oder mehrere Verbindungen aus der Gruppe bestehend aus 1-Hydroxy-2-pentanon, Furfurylalkohol und Furfural,
(f) Bereitstellen eines zweiten heterogenen Katalysators wie oben definiert,
(h') Kontaktieren des in Schritt (e) gebildeten Gemischs in Gegenwart von Wasserstoff und optional eines Inertgases mit dem gemäß Schritt (f) bereitgestellten zweiten heterogenen Katalysator,
(i) Umsetzen von Verbindungen aus der Gruppe bestehend aus 1-Hydroxy-2-pentanon, Furfurylalkohol und Furfural aus dem in Schritt (e) gebildeten Gemisch in Gegenwart des zweiten heterogenen Katalysators mit Wasserstoff unter Bildung von 1,2-Pentandiol,
(j) optional destillatives Abtrennen des 1,2-Pentandiol.

In einer zweiten bevorzugten Variante umfasst das erfindungsgemäße Verfahren die Schritte:
(a) Bereitstellen eines Edukts umfassend eine oder beide Verbindungen aus der Gruppe bestehend aus Furfurylalkohol und Furfural,
(b) Bereitstellen eines ersten heterogenen Katalysators wie oben definiert,
(c) optional Bereitstellen eines oder mehrerer Verdünnungsmittel,
(d) Herstellen einer Mischung umfassend die in den Schritten (a) und (b) sowie optional (c) bereitgestellten Komponenten und Kontaktieren dieser Mischung mit Wasserstoff
(e) in der in Schritt (d) hergestellten Mischung Umsetzen des Edukts mit Wasserstoff in Gegenwart des ersten heterogenen Katalysators unter Bildung eines Gemisches enthaltend 1,2-Pentandiol und eine oder mehrere Verbindungen aus der Gruppe bestehend aus 1-Hydroxy-2-pentanon, Furfurylalkohol und Furfural,
(f) Bereitstellen eines zweiten heterogenen Katalysators wie oben definiert,
(g) optional Bereitstellen eines oder mehrerer Verdünnungsmittel,
(h) Herstellen einer Mischung umfassend das in Schritt (e) gebildeten Gemisch und die in den Schritten (f) und optional (g) bereitgestellten Komponenten und Kontaktieren dieser Mischung mit Wasserstoff
(i) Umsetzen von Verbindungen aus der Gruppe bestehend aus 1-Hydroxy-2-pentanon, Furfurylalkohol und Furfural aus dem in Schritt (e) gebildeten Gemisch in Gegenwart des zweiten heterogenen Katalysators mit Wasserstoff unter Bildung von 1,2-Pentandiol,
(j) optional destillatives Abtrennen des 1,2-Pentandiol.

In einer dritten bevorzugten Variante umfasst das erfindungsgemäße Verfahren die Schritte:
(a) Bereitstellen eines Edukts umfassend eine oder beide Verbindungen aus der Gruppe bestehend aus Furfurylalkohol und Furfural,
(b) Bereitstellen eines ersten heterogenen Katalysators wie oben definiert,
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator,
(e) in der in Schritt (d') hergestellten Mischung Umsetzen des Edukts mit Wasserstoff in Gegenwart des ersten heterogenen Katalysators unter Bildung eines Gemisches enthaltend 1,2-Pentandiol und eine oder mehrere Verbindungen aus der Gruppe bestehend aus 1-Hydroxy-2-pentanon, Furfurylalkohol und Furfural,
(f) Bereitstellen eines zweiten heterogenen Katalysators wie oben definiert,
(g) optional Bereitstellen eines oder mehrerer Verdünnungsmittel,
(h) Auskondensieren organischer Komponenten des in Schritt (e) gebildeten Gemischs und Herstellen einer Mischung umfassend aus dem in Schritt (e) gebildeten Gemisch auskondensierte organische Komponenten und die in den Schritten (f) und optional (g) bereitgestellten Komponenten und Kontaktieren dieser Mischung mit Wasserstoff
(i) Umsetzen von Verbindungen aus der Gruppe bestehend aus 1-Hydroxy-2-pentanon, Furfurylalkohol und Furfural aus dem in Schritt (e) gebildeten Gemisch in Gegenwart des zweiten heterogenen Katalysators mit Wasserstoff unter Bildung von 1,2-Pentandiol,
(j) optional destillatives Abtrennen des 1,2-Pentandiol.

In einer vierten bevorzugten Variante umfasst das erfindungsgemäße Verfahren die Schritte:
(a) Bereitstellen eines Edukts umfassend eine oder beide Verbindungen aus der Gruppe bestehend aus Furfurylalkohol und Furfural,
(b) Bereitstellen eines ersten heterogenen Katalysators wie oben definiert,
(c) optional Bereitstellen eines oder mehrerer Verdünnungsmittel,
(d) Herstellen einer Mischung umfassend die in den Schritten (a) und (b) sowie optional (c) bereitgestellten Komponenten und Kontaktieren dieser Mischung mit Wasserstoff
(e) in der in Schritt (d) bzw. (d') hergestellten Mischung Umsetzen des Edukts mit Wasserstoff in Gegenwart des ersten heterogenen Katalysators unter Bildung eines Gemisches enthaltend 1,2-Pentandiol und eine oder mehrere Verbindungen aus der Gruppe bestehend aus 1-Hydroxy-2-pentanon, Furfurylalkohol und Furfural,
(f) Bereitstellen eines zweiten heterogenen Katalysators wie oben definiert,
(h') Kontaktieren des in Schritt (e) gebildeten Gemischs in Gegenwart von Wasserstoff und optional eines Inertgases mit dem gemäß Schritt (f) bereitgestellten zweiten heterogenen Katalysator,
(i) Umsetzen von Verbindungen aus der Gruppe bestehend aus 1-Hydroxy-2-pentanon, Furfurylalkohol und Furfural aus dem in Schritt (e) gebildeten Gemisch in Gegenwart des zweiten heterogenen Katalysators mit Wasserstoff unter Bildung von 1,2-Pentandiol,
(j) optional destillatives Abtrennen des 1,2-Pentandiol.

Das erfindungsgemäße Verfahren, insbesondere in seinen bevorzugten Varianten und der oben beschriebenen vorteilhaften Weiterentwicklung, führt zu einem Produktgemisch, in dem 1,2-Pentandiol regelmäßig das Hauptprodukt darstellt.

Das nachfolgende Reaktionsschema veranschaulicht die Umsetzung von Furfural bzw. Furfurylalkohol in einem erfindungsgemäßen Verfahren über das Zwischenprodukt 1-Hydroxy-2-pentanon zu dem gewünschten Hauptprodukt 1,2-Pentandiol und den dabei regelmäßig erhaltenen Nebenprodukten.

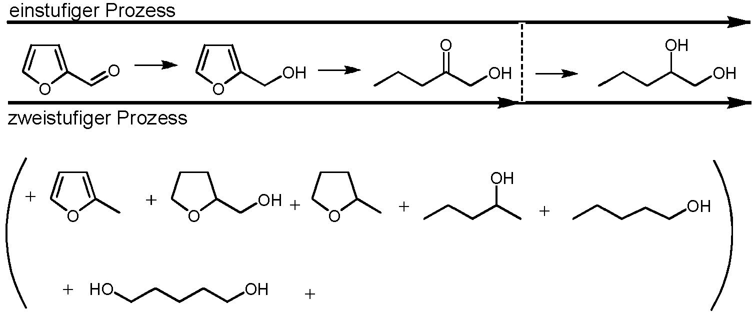

Wird Furfural als Edukt eingesetzt, so wird dies zunächst zu Furfurylalkohol umgesetzt.

Wird das erfindungsgemäße Verfahren als einstufiger Prozess (umfassend einen Umsetzungsschritt in Gegenwart eines ersten heterogenen Katalysators wie oben beschrieben, jedoch keinen weiteren Umsetzungsschritt in Gegenwart eines zweiten heterogenen Katalysators wie oben beschrieben), so entstehen neben dem Zielprodukt 1,2-Pentandiol die im unteren Teil des Schemas in Klammern angegebenen Nebenprodukte sowie 1-Hydroxy-2-pentanon. Diese Variante wird im obigen Schema symbolisiert durch den durchgehenden Pfeil mit der Beschriftung "einstufiger Prozess".

Wird das erfindungsgemäße Verfahren gemäß der vorteilhaften Weiterentwicklung als zweistufiger Prozess (umfassend einen Umsetzungsschritt in Gegenwart eines ersten heterogenen Katalysators wie oben beschrieben und einen weiteren Umsetzungsschritt in Gegenwart eines zweiten heterogenen Katalysators wie oben beschrieben), so entstehen neben dem Zielprodukt 1,2-Pentandiol die im unteren Teil des Schemas in Klammern angegebenen Nebenprodukte, während bei der Umsetzung in Gegenwart des ersten heterogenen Katalysators gebildetes 1-Hydroxy-2-pentanon bei der Umsetzung in Gegenwart des zweiten heterogenen Katalysators weitgehend zu 1,2-Pentandiol umgesetzt wird. Diese Variante wird im obigen Schema symbolisiert durch den zweiteiligen Pfeil mit der Beschriftung "zweistufiger Prozess".

Vorzugsweise erfolgt die Reaktionsführung in der Weise, dass zumindest 80 Gew.-%, bevorzugt zumindest 90 Gew.-%, weiter bevorzugt zumindest 95 Gew.-% des eingesetzten Furfurals und/oder Furfurylalkohols umgesetzt werden.

Vorzugsweise erfolgt die Reaktionsführung in der Weise, dass das nach dem ein- oder zweistufigen Verfahren vorliegende Reaktionsgemisch zumindest 30 Gew.-% an 1,2-Pentandiol enthält, bevorzugt zumindest 50 Gew.-%, weiter bevorzugt zumindest 70 Gew.-%, besonders bevorzugt zumindest 80 Gew.-%, bezogen auf die Gesamtmenge der entstandenen Produkte. Dabei beziehen sich die Anteile Y an 1,2-Pentandiol bzw. 1-Hydroxy-2-Pentanon in dem Reaktionsgemisch auf die Gesamtstoffmenge der aus Furfurylalkohol oder Furfural entstandenen Produkte. Dementsprechend nicht zu berücksichtigen sind für die Ermittlung des Anteils Y an 1,2-Pentandiol eventuell in dem Reaktionsgemisch nach Beendigung der Umsetzung noch vorhandene Mengen an nicht umgesetzten Furfurylalkohol bzw. Furfural sowie die Mengen an Katalysator(en) und Verdünnungsmittel(n). Die Anteile Y an 1,2-Pentandiol bzw. 1-Hydroxy-2-Pentanon in dem Reaktionsgemisch beziehen sich insbesondere auf die Gesamtstoffmenge an 1-Pentanol, 2-Pentanol, Pentan, Tetrahydrofurfurylalkohol, 1-Hydroxy-2-pentanon, Methylfuran, Methyltetrahydrofuran, 1,2-Pentandiol und 1,5-Pentandiol in dem Reaktionsgemisch nach erfolgter Umsetzung.

Die vorliegende Erfindung betrifft auch die Verwendung eines heterogenen Katalysators umfassend
(i) ein oder mehrere Metalle aus der Gruppe bestehend aus Platin, Rhodium, Nickel und Iridium in metallischer Form und/oder eine oder mehrere Verbindungen von Metallen aus der Gruppe bestehend aus Platin, Rhodium, Nickel und Iridium,
   sowie
(ii) ein oder mehrere Trägermaterialien
   zur Hydrogenolyse eines Edukts umfassend eine oder mehrere Verbindungen aus der Gruppe bestehend aus Furfurylalkohol, Furfural und 1-Hydroxy-2-Pentanon oder zur Umsetzung eines Edukts umfassend eine oder mehrere Verbindungen aus der Gruppe bestehend aus Furfurylalkohol, Furfural und 1-Hydroxy-2-Pentanon zu einem hydrierten Produkt.

Hinsichtlich bevorzugter Ausgestaltungen und bevorzugter Einsatzbedingungen des erfindungsgemäß zu verwendenden Katalysators sei auf die vorstehenden Ausführungen verwiesen.

Die Erfindung betrifft ferner die Verwendung eines Platin-Katalysators umfassend (i) Platin und/oder eine oder mehrere Platinverbindungen und (ii) ein oder mehrere Trägermaterialien zur Hydrogenolyse von Furfurylalkohol.

Die Erfindung betrifft insbesondere die Verwendung eines Platin-Katalysators ausgewählt aus der Gruppe bestehend aus Platin auf Aktivkohle, Platin auf Aluminiumoxid und Platindioxid auf Aluminiumoxid zur Hydrogenolyse von Furfurylalkohol.

Das durch das erfindungsgemäße Verfahren erhaltene 1,2-Pentandiol lässt sich im Bedarfsfalle durch sich anschließende einfache Schritte weiter reinigen, beispielsweise mittels Destillation oder Rektifikation. Es ist dann weitgehend oder vollkommen farblos und weitgehend oder vollkommen geruchlos und - auch wegen der geruchlichen Qualität - für den Einsatz in der Kosmetik geeignet.

In den nachfolgenden Beispielen beziehen sich alle Angaben auf das Gewicht, sofern nichts anderes angegeben ist.

### Beispiel 1 (erfindungsgemäß)

100 g (1,02 mol) Furfurylalkohol wurden in 200 g Ethanol gelöst, mit 10 g Katalysator (5 Gew.-% Platindioxid auf gamma-Aluminiumoxid, entsprechend 0,5 g Platindioxid) versetzt und bei 0 bis 5 °C und einem Wasserstoffdruck von 0,1 MPa (1 bar) hydriert. Nach 5 Stunden war die Wasserstoffaufnahme beendet. Der Katalysator wurde abfiltriert, das Lösungsmittel Ethanol und schließlich das Produktgemisch destilliert. Es wurden 105 g eines Destillats, welches folgende Zusammensetzung aufwies:
2% 2-Pentandiol
2% 1-Pentandiol
15% Tetrahydrofurfurol
80% 1,2-Pentandiol
1% 1,5-Pentandiol

Ausbeute: 84 g (0,81 mol) 1,2-Pentandiol (entsprechend 80% der Theorie).

Es wurden die Destillate aus sechs Hydrieransätzen vereinigt (insgesamt 630 g) und an einer 1m-Füllkörperkolonne fraktioniert destilliert. Dabei wurden als Hauptfraktion 475 g farbloses 1,2-Pentandiol mit einer Reinheit von 99,9% erhalten.

Das erhaltene 1,2-Pentandiol war farb- und geruchlos.

Der in Beispiel 1 eingesetzte heterogene Platin-Katalysator wurde hergestellt, indem 1,8 g Hexachloroplatinsäure in Wasser gelöst und mittels eines incipient-wetness-Verfahrens auf 10 g des Trägermaterials gamma-Aluminiumoxid aufgebracht wurden. Der so erhaltene Feststoff wurde bei einer Temperatur im Bereich von 310 bis 320 °C in eine NaNO₃-Schmelze eingebracht, die Temperatur des resultierenden Gemisches allmählich bis auf 500 °C erhöht und das Gemisch anschließend 1 h bei 500 °C gehalten.

### Beispiel 2 (Vergleichsbeispiel I)

In Anlehnung an Journal of American Chemical Society 71, 415 (1949) bzw. dort zitierter Literaturstelle Journal of American Chemical Society 67, 272 (1945) wurden 100 g (1,02 mol) Furfurylalkohol in 200 g Essigsäure gelöst, mit 4 g Platindioxid (ohne Trägermaterial; Lieferant: Acros) versetzt und bei 0 bis 5 °C und einem Wasserstoffdruck von 0,1 MPa (1 bar) hydriert. Nach 10 Stunden war die Wasserstoffaufnahme beendet, der Katalysator wurde abfiltriert und das Lösungsmittel abdestilliert. Anschließend wurde der Destillationsrückstand in 500 g Methyl-tert.-butylether aufgenommen und mit 40 g (0,74 mol) Natriummethylat gerührt, um die als Mono- bzw. Diacetat vorliegenden Diole zu deacetylieren. Nach der Zugabe von 50 g Wasser wurde mit halbkonzentrierter Salzsäure neutralisiert, die Phasen getrennt und das Lösungsmittel entfernt. Nach Destillation wurden 80 g einer öligen Flüssigkeit mit folgender Zusammensetzung erhalten:
26% 2-Pentandiol
10% 1-Pentandiol
35% Tetrahydrofurfurol
26% 1,2-Pentandiol
2% 1,5-Pentandiol
Ausbeute: 21 g (0,20 mol) 1,2-Pentandiol (entsprechend 20% der Theorie).

### Beispiel 3 (Vergleichsbeispiel II)

In Anlehnung an Journal of American Chemical Society 67, 272 (1945) wurden 5 g (0,05 mol) Furfurylalkohol in 50 ml Essigsäure gelöst, mit 0,2 g Platindioxid versetzt und bei 20 bis 25 °C und einem Wasserstoffdruck von 0,1 MPa (1 bar) hydriert.

Das Resultat entsprach dem des Beispiels 2 (Vergleichsbeispiel I).

### Beispiele 4-7 (erfindungsgemäß)

In den Rohrreaktor einer kontinuierlichen Gasphasenapparatur, wurden 3 g eines granulierten heterogenen Katalysators umfassend (i) Platin und (ii) Al₂O₃ angeordnet. Die Konzentration an (i) Platin beträgt 10 Gew.-% bezogen auf das Gesamtgewicht aller Bestandteile des Katalysators. Die Apparatur umfasst einem Sättiger befüllt mit Furfurylalkohol (FA), Einleitungsrohre für Wasserstoff und ein Inertgas, Gasdosiereinheiten, einem Rohrreaktor und eine Kondensationseinheit.

Der Volumenstrom an Furfurylalkohol wurde über die Temperatur des Sättigers und die Dosierung des Wasserstoffstroms und gegebenenfalls einen zusätzlichen Inertgasstrom gesteuert. Das gasförmige Gemisch umfassend Furfurylalkohol und Wasserstoff wurde durch den Rohrreaktor geleitet und organische Komponenten sowie Wasser aus dem Produktgasstrom in einer Kondensationseinheit in den flüssigen Aggregatzustand überführt. In regelmäßigen Abständen wurden Proben entnommen und mittels verschiedener analytischer Methoden die Zusammensetzung des Kondensats analysiert. Die Reaktionsbedingungen sowie der Umsatz X bezogen auf Furfurylalkohol und die Anteile Y (wie oben definiert) von 1-Hydroxy-2 Pentanon und 1,2-Pentandiol an der Gesamtstoffmenge der aus Furfurylalkohol gebildeten Produkte sind in Tabelle 1 aufgeführt.

### Beispiel 8 (erfindungsgemäß)

Es wurde die Versuchsanordnung der Beispiele 4-7 benutzt, wobei im Rohrreaktor 3 g eines heterogenen Katalysators umfassend (i) Platin und (ii) Al₂O₃ angeordnet wurden. Die Konzentration an (i) Platin beträgt 5 Gew.-% bezogen auf das Gesamtgewicht aller Bestandteile des Katalysators. Die weitere Vorgehensweise entspricht den Beispielen 4-7. Die Reaktionsbedingungen sowie der Umsatz X bezogen auf Furfurylalkohol und die Anteile Y (wie oben definiert) von 1-Hydroxy-2 Pentanon und 1,2-Pentandiol an der Gesamtstoffmenge der aus Furfurylalkohol gebildeten Produkte sind in Tabelle 1 aufgeführt.

**Tabelle 1**

| Beispiel Nr. | Fluss FA [g/h] | C_{FA} im Gasstrom [mol-%] | T_{Sätt} [°C] | T_{Reakt} [°C] | H₂-Fluss [mln/min] | Probenahmezeit [min] | X FA [%]* | Y 1,2-PD [%]* | Y 1-Hydroxy-2-pentanon [%]* |
|---|---|---|---|---|---|---|---|---|---|
| 4 | 0,619 | 3,78 | 90 | 230 | 60 | 123 | 98,3 | 38,0 | 54 |
| 5 | 0,268 | 3,29 | 90 | 230 | 30 | 119 | 99,8 | 47,0 | 51 |
| 6 | 0,450 | 1,87 | 110 | 230 | 30/2 bar H₂-Überdruck | 303 | 99,5 | 60,9 | 17,8 |
| 7 | 0,159 | 1,98 | 110 | 190 | 30/4 bar H₂-Überdruck | 295 | 99,9 | 69,7 | 3,0 |
| 8 | 0,387 | 4,68 | 90 | 220 | 30 | 331 | 99,6 | 37,9 | 40,1 |

| | | | | | | | | | |
|---|---|---|---|---|---|---|---|---|---|
| *Die prozentualen Angaben beziehen sich auf Stoffmengen. | | | | | | | | | |

### Beispiel 9 (erfindungsgemäß)

0,5 g eines Kondensats aus einer Reaktion in der Gasphase wie oben beschrieben wurden in 25 ml Ethanol in Gegenwart eines Katalysators umfassend (i) Platin und (ii) Al₂O₃ über 4 Stunden bei Raumtemperatur unter 5 bar H₂ zur Reaktion gebracht. Das Kondensat enthielt 0,1% Furfurylalkohol, 35 % 1,2-Pentandiol und 36% 1-Hydroxy-2-pentanon. Die Konzentration an (i) Platin beträgt 10 Gew.-% bezogen auf das Gesamtgewicht aller Bestandteile des Katalysators sowie 2 mol-% bezogen auf die Stoffmenge an 1-Hydroxy-2-Pentanon im Kondensat.

Die Zusammensetzung des Reaktionsgemisches wurde nach Abfiltrieren des Katalysators mittels Gaschromatographie/Massenspektroskopie (GC/MS) ermittelt. Der Umsatz X bezogen auf Furfurylalkohol und die Anteile Y (wie oben definiert) von 1-Hydroxy-2 Pentanon und 1,2-Pentandiol an der Gesamtstoffmenge der aus Furfurylalkohol gebildeten Produkte sind in Tabelle 2 aufgeführt.

### Beispiel 10 (erfindungsgemäß)

0,5 g eines Kondensats aus einer Reaktion in der Gasphase wie oben beschrieben wurden in 25 ml Ethanol in Gegenwart eines Katalysators umfassend (i) Rhodium und (ii) Al₂O₃ über 4 Stunden bei Raumtemperatur unter 2 MPa (20 bar) H₂ zur Reaktion gebracht. Das Kondensat enthielt 0,1% Furfurylalkohol, 35 % 1,2-Pentandiol und 36% 1-Hydroxy-2-pentanon. Die Konzentration an (i) Rhodium beträgt 5 Gew.-% bezogen auf das Gesamtgewicht aller Bestandteile des Katalysators sowie 2 mol-% bezogen auf die Stoffmenge an 1-Hydroxy-2-Pentanon im Kondensat.

Die Zusammensetzung des Reaktionsgemisches wurde nach Abfiltrieren des Katalysators mittels GC/MS ermittelt. Der Umsatz X bezogen auf Furfurylalkohol und die Anteile Y (wie oben definiert) von 1-Hydroxy-2 Pentanon und 1,2-Pentandiol an der Gesamtstoffmenge der aus Furfurylalkohol gebildeten Produkte sind in Tabelle 2 aufgeführt.

**Tabelle 2**

| Beispiel Nr. | X FA [%]* | Y 1,2-PD [%]* | Y 1-Hydroxy-2-pentanon [%]* |
|---|---|---|---|
| 9 | 100 | 49 | 14 |
| 10 | 100 | 44 | 14 |

| | | | |
|---|---|---|---|
| *Die prozentualen Angaben beziehen sich auf Stoffmengen. | | | |

### Beispiel 11 (erfindungsgemäß)

0,5g 1-Hydroxy-2-pentanon (94%ig) wurden in 25 ml Ethanol in Gegenwart eines Katalysators umfassend (i) Platin und (ii) Al₂O₃ über 4 Stunden bei Raumtemperatur unter 0,5 MPa (5 bar) H₂ zur Reaktion gebracht. Die Konzentration an (i) Platin beträgt 10 Gew.-% bezogen auf das Gesamtgewicht aller Bestandteile des Katalysators sowie 2 mol-% bezogen auf die Stoffmenge an 1-Hydroxy-2-Pentanon.

Die Zusammensetzung des Reaktionsgemisches wurde nach Abtrennen des Katalysators mittels GC/MS ermittelt. Der Umsatz X bezogen auf 1-Hydroxy-2-pentanon und der Anteil Y des 1,2-Pentandiol an der Gesamtstoffmenge der aus 1-Hydroxy-2-pentanon gebildeten Produkte sind in Tabelle 3 aufgeführt.

### Beispiel 12 (erfindungsgemäß)

0,5g 1-Hydroxy-2-pentanon (94%ig) wurden in 25 ml Ethanol in Gegenwart eines Katalysators umfassend (i) Platin und (ii) Al₂O₃ über 4 Stunden bei Raumtemperatur unter 0,5 MPa (5 bar) H₂ zur Reaktion gebracht. Die Konzentration an (i) Platin beträgt 1 Gew.-% bezogen auf das Gesamtgewicht aller Bestandteile des Katalysators sowie 2 mol-% bezogen auf die Stoffmenge an 1-Hydroxy-2-Pentanon.

Die Zusammensetzung des Reaktionsgemisches wurde nach Abtrennen des Katalysators mittels GC/MS ermittelt. Der Umsatz X bezogen auf 1-Hydroxy-2-pentanon und der Anteil Y des 1,2-Pentandiol an der Gesamtstoffmenge der aus 1-Hydroxy-2-pentanon gebildeten Produkte sind in Tabelle 3 aufgeführt.

### Beispiel 13 (Vergleichsbeispiel III)

0,5g 1-Hydroxy-2-pentanon (94%ig) wurden in 25 ml Ethanol in Gegenwart eines Katalysators umfassend (i) Ruthenium und (ii) Al₂O₃ über 6 Stunden bei Raumtemperatur unter 5 MPa (50 bar) H₂ zur Reaktion gebracht. Die Konzentration an (i) Ruthenium beträgt 5 Gew.-% bezogen auf das Gesamtgewicht aller Bestandteile des Katalysators sowie 2 mol-% bezogen auf die Stoffmenge an 1-Hydroxy-2-Pentanon.

Die Zusammensetzung des Reaktionsgemisches wurde nach Abtrennen des Katalysators mittels GC/MS ermittelt. Der Umsatz X bezogen auf 1-Hydroxy-2-pentanon und der Anteil Y des 1,2-Pentandiol an der Gesamtstoffmenge der aus 1-Hydroxy-2-pentanon gebildeten Produkte sind in Tabelle 3 aufgeführt.

**Tabelle 3**

| Beispiel Nr. | X 1-Hydroxy-2-pentanon [%]* | Y 1,2-PD [%]* |
|---|---|---|
| 11 | 69 | 69 |
| 12 | 99 | 80 |
| 13 | 98 | 96 |

| | | |
|---|---|---|
| *Die prozentualen Angaben beziehen sich auf Stoffmengen. | | |

## Patentansprüche

1. Verfahren zum Herstellen von 1,2-Pentandiol, umfassend den Schritt
- Umsetzung eines Edukts umfassend eine oder beide Verbindungen aus der Gruppe bestehend aus Furfurylalkohol und Furfural in Gegenwart eines ersten heterogenen Katalysators mit Wasserstoff unter Bildung eines Gemisches enthaltend 1,2-Pentandiol und gegebenenfalls eine oder mehrere Verbindungen aus der Gruppe bestehend aus 1-Hydroxy-2-pentanon, Furfurylalkohol und Furfural, wobei bei der Umsetzung in Gegenwart des ersten heterogenen Katalysators der Wasserstoffpartialdruck im Bereich von 0,1 bis 0,8 MPa (1 bis 8 bar liegt),
wobei der erste heterogene Katalysator
(i) ein oder mehrere Metalle aus der Gruppe bestehend aus Platin, Rhodium, Nickel und Iridium in metallischer Form und/oder eine oder mehrere Verbindungen von Metallen aus der Gruppe bestehend aus Platin, Rhodium, Nickel und Iridium,
sowie
(ii) ein oder mehrere Trägermaterialien
umfasst.

2. Verfahren nach Anspruch 1, wobei der erste heterogene Katalysator (i) Platin in metallischer Form und/oder eine oder mehrere Platin(IV)-Verbindungen umfasst, wobei der erste heterogene Katalysator bevorzugt ausgewählt ist aus der Gruppe bestehend aus
- Katalysatoren umfassend (i) Platin in metallischer Form und (ii) Aktivkohle, insbesondere Platin auf Aktivkohle,
- Katalysatoren umfassend (i) Platin in metallischer Form und (ii) Aluminiumoxid, insbesondere Platin auf Aluminiumoxid,
- Katalysatoren umfassend (i) Platin in metallischer Form und (ii) Siliciumdioxid, insbesondere Platin auf Siliciumdioxid,
- Katalysatoren umfassend (i) Platin in metallischer Form und (ii) Siliciumcarbid, insbesondere Platin auf Siliciumcarbid,
- Katalysatoren umfassend (i) Platin(IV)-oxid und (ii) Aluminiumoxid, insbesondere Platindioxid auf Aluminiumoxid.

3. Verfahren nach einem der vorangehenden Ansprüche, wobei
- im ersten heterogenen Katalysator die Gesamtkonzentration an (i) Platin, Rhodium, Nickel und Iridium in metallischer Form und in Form von in Verbindungen dieser Metalle enthaltenem Platin, Rhodium, Nickel und Iridium im Bereich von 0,1 Gew.-% bis 50 Gew.-%, bevorzugt von 0,5 Gew.-% bis 20 Gew.-%, weiter bevorzugt von 1 bis 10 Gew.-%, besonders bevorzugt von 1 Gew.-% bis 5 Gew.-% liegt, bezogen auf das Gesamtgewicht aller Bestandteile des ersten heterogenen Katalysators
und/oder
- die Menge an erstem heterogenem Katalysator im Bereich von 0,1 bis 20 Gew.-% liegt, bevorzugt im Bereich von 0,25 bis 15 Gew.-%, weiter bevorzugt im Bereich von 0,5 bis 12 Gew.-%, bezogen auf die eingesetzte Menge an Furfurylalkohol, wobei der erste heterogene Katalysator (i) Platin in metallischer Form oder eine oder mehrere Verbindungen von Platin enthält
und/oder
- die Gesamtkonzentration an (i) Platin, Rhodium, Nickel und Iridium in metallischer Form und in Form von in Verbindungen dieser Metalle enthaltenem Platin, Rhodium, Nickel und Iridium im ersten heterogenen Katalysator im Bereich von 0,01 bis 10 Mol.-% liegt, bevorzugt im Bereich von 0,05 bis 5 Mol.-%, weiter bevorzugt im Bereich von 0,1 bis 2 Mol.-%, bezogen auf die eingesetzte Gesamtstoffmenge an Furfurylalkohol und Furfural.

4. Verfahren nach einem der vorangehenden Ansprüche, wobei bei der Umsetzung in Gegenwart des ersten heterogenen Katalysators der Wasserstoffpartialdruck im Bereich von 0,1 bis 0,4 MPa (1 bis 4 bar).

5. Verfahren nach einem der vorangehenden Ansprüche, wobei bei der Umsetzung in Gegenwart des ersten heterogenen Katalysators das molare Verhältnis zwischen Wasserstoff und der Gesamtmenge an Furfurylalkohol und Furfural 1:1 oder mehr beträgt und bevorzugt im Bereich von 4:1 bis 100:1 liegt, besonders bevorzugt im Bereich von 5:1 bis 20:1.

6. Verfahren nach einem der vorangehenden Ansprüche, wobei die Umsetzung in Gegenwart des ersten heterogenen Katalysators in einer Gasphase erfolgt, bevorzugt bei einer Temperatur im Bereich von 100 °C bis 250 °C, weiter bevorzugt im Bereich von 150 °C bis 240 °C, besonders bevorzugt im Bereich von 170 °C bis 230 °C.

7. Verfahren nach Anspruch 6, wobei die Umsetzung in Gegenwart des ersten heterogenen Katalysators in einem Reaktor erfolgt, der kontinuierlich von einem Gasstrom durchströmt wird, der beim Eintritt in den Reaktor das besagte Edukt, Wasserstoff und optional ein Inertgas umfasst,
wobei
- die Strömungsrate dieses Gasstroms bezogen auf das Volumen des ersten heterogenen Katalysators (Gas hourly space velocity GHSV) 500 h⁻¹ bis 5000 h⁻¹, besonders bevorzugt 900 h⁻¹ bis 3600 h⁻¹ beträgt
und/oder
- die Gesamtkonzentration an Furfurylalkohol und Furfural in dem in den Reaktor eintretenden Gasstrom 1 Mol-% bis 15 Mol-%, bevorzugt 3 Mol-% bis 10 Mol-% beträgt.

8. Verfahren nach einem der Ansprüche 1 bis 5, wobei
- die Umsetzung in Gegenwart des ersten heterogenen Katalysators in einer flüssigen Phase erfolgt
und/oder
- bei einer Temperatur im Bereich von -20 °C bis +100 °C, bevorzugt von -20 °C bis + 50 °C, weiter bevorzugt im Bereich von -5 bis + 50 °C, noch weiter bevorzugt im Bereich von -5 °C bis +30 °C, besonders bevorzugt im Bereich von 0 °C bis +30 °C, ganz besonders bevorzugt im Bereich von 0 °C bis +10 °C.

9. Verfahren nach Anspruch 8, wobei die flüssige Phase ein oder mehrere, vorzugsweise organische, Verdünnungsmittel mit einem pKₛ-Wert bei 25 °C von größer oder gleich 6 umfasst, bevorzugt mit einem pKₛ-Wert bei 25 °C von größer oder gleich 8, weiter bevorzugt mit einem pKₛ-Wert bei 25 °C von größer oder gleich 10, besonders bevorzugt mit einem pKₛ-Wert bei 25 °C von größer oder gleich 12 wobei bevorzugt
- das Verdünnungsmittel einen oder mehrere Alkohole mit 1 bis 4 Kohlenstoffatomen umfasst oder daraus besteht, vorzugsweise ausgewählt aus der Gruppe bestehend aus Methanol, Ethanol, n-Propanol, Isopropanol und deren Mischungen,
und/oder
- die Gesamtmenge der Verdünnungsmittel im Bereich von 25 bis 1000 Gew.-% liegt, bevorzugt im Bereich von 50 bis 500 Gew.-%, weiter bevorzugt im Bereich von 100 bis 300 Gew.-%, bezogen auf die eingesetzte Gesamtmenge an Furfurylalkohol oder die Gesamtmenge der Verdünnungsmittel im Bereich von 25 bis 1000 Gew.-% liegt, bevorzugt im Bereich von 50 bis 500 Gew.-%, weiter bevorzugt im Bereich von 100 bis 300 Gew.-%, bezogen auf die eingesetzte Gesamtmenge an Furfurylalkohol und Furfural.

10. Verfahren nach einem der vorangehenden Ansprüche, umfassend folgende Schritte:
(a) Bereitstellen eines Edukts umfassend eine oder beide Verbindungen aus der Gruppe bestehend aus Furfurylalkohol und Furfural;
(b) Bereitstellen eines ersten heterogenen Katalysators wie in Anspruch 1 definiert;
(c) optional Bereitstellen eines oder mehrerer Verdünnungsmittel;
(d) Herstellen einer Mischung umfassend die in den Schritten (a) und (b) sowie optional (c) bereitgestellten Komponenten und Kontaktieren dieser Mischung mit Wasserstoff
oder
(d') Herstellen einer Mischung umfassend das in Schritt (a) bereitgestellte Edukt und Wasserstoff sowie optional ein Inertgas und Kontaktieren dieser Mischung mit dem gemäß Schritt (b) bereitgestellten ersten heterogenen Katalysator;
(e) in der in Schritt (d) bzw. (d') hergestellten Mischung Umsetzen des Edukts mit Wasserstoff in Gegenwart des ersten heterogenen Katalysators unter Bildung eines Gemisches enthaltend 1,2-Pentandiol und gegebenenfalls eine oder mehrere Verbindungen aus der Gruppe bestehend aus 1-Hydroxy-2-pentanon, Furfurylalkohol und Furfural;
(f) optional destillative Abtrennung des 1,2-Pentandiol.

11. Verfahren nach einem der vorhergehenden Ansprüche, weiter umfassend den Schritt
- Umsetzung von Verbindungen aus der Gruppe bestehend aus 1-Hydroxy-2-pentanon, Furfurylalkohol und Furfural aus dem in einem Verfahren nach einem der vorhergehenden Ansprüche gebildeten Gemisch in Gegenwart eines zweiten heterogenen Katalysators mit Wasserstoff unter Bildung von 1,2-Pentandiol,
wobei der zweite heterogene Katalysator
(i') ein oder mehrere Metalle aus der Gruppe bestehend aus Platin, Rhodium, Ruthenium, Nickel, Palladium und Iridium in metallischer Form und/oder eine oder mehrere Verbindungen von Metallen aus der Gruppe bestehend aus Platin, Rhodium, Ruthenium, Nickel, Palladium und Iridium,
sowie
(ii') ein oder mehrere Trägermaterialien
umfasst.

12. Verfahren nach einem der vorangehenden Ansprüche, wobei das Trägermaterial (ii) des ersten heterogenen Katalysators und/oder das Trägermaterial (ii') des zweiten heterogenen Katalysators bei 25 °C und 0,1013 MPa (1013 mbar), vorzugsweise auch bei 230 °C und 0,1013 MPa (1013 mbar), fest ist, wobei vorzugsweise das Trägermaterial (ii) und/oder das Trägermaterial (ii') ausgewählt ist aus der Gruppe bestehend aus Aktivkohle, Kieselsäure, Siliciumdioxid, Siliciumcarbid, Aluminiumoxid, Zirconiumdi-oxid, Titandioxid, Niobtrioxid, Ceriumdioxid und deren Gemischen.

13. Verfahren nach einem der Ansprüche 11 und 12, wobei
- wobei der erste und der zweite heterogene Katalysator die gleiche Zusammensetzung haben,
und/oder
- im zweiten heterogenen Katalysator die Gesamtkonzentration an (i) Platin, Rhodium, Ruthenium, Nickel, Palladium und Iridium in metallischer Form und in Form von in Verbindungen dieser Metalle enthaltenem Platin, Rhodium, Ruthenium, Nickel, Palladium und Iridium im Bereich von 0,1 Gew.-% bis 20 Gew.-% liegt, bevorzugt von 0,5 Gew.-% bis 10 Gew.-%, bezogen auf das Gesamtgewicht aller Bestandteile des zweiten heterogenen Katalysators.

14. Verfahren nach einem der Ansprüche 11 bis 13, wobei die Umsetzung in Gegenwart des zweiten heterogenen Katalysators in einer Gasphase erfolgt, bevorzugt bei einer Temperatur im Bereich von 25 °C bis 240 °C, besonders bevorzugt im Bereich von 100 °C bis 130 °C,
wobei bevorzugt die Umsetzung in Gegenwart des zweiten heterogenen Katalysators bevorzugt in einem Reaktor erfolgt, der kontinuierlich von einem Gasstrom durchströmt wird, wobei dieser Gasstrom beim Eintritt in den Reaktor das in einem Verfahren nach einem der Ansprüche 1 bis 10 gebildete Gemisch sowie Wasserstoff und optional ein Inertgas umfasst, wobei
- die Strömungsrate dieses Gasstroms bezogen auf das Volumen des zweiten heterogenen Katalysators (Gas hourly space velocity GHSV) 500 h⁻¹ bis 5000 h⁻¹, bevorzugt 900 h⁻¹ bis 3600 h⁻¹ beträgt
und/oder
- die Gesamtkonzentration an 1-Hydroxy-2-pentanon, Furfurylalkohol und Furfural in dem in den Reaktor eintretenden Gasstrom 1 Mol-% bis 15 Mol-%, bevorzugt 3 Mol-% bis 10 Mol-% beträgt.

15. Verfahren nach einem der Ansprüche 11 bis 13, wobei die Umsetzung in Gegenwart des zweiten heterogenen Katalysators in einer flüssigen Phase erfolgt wobei bevorzugt
- die Temperatur im Bereich von -20 °C bis +150 °C liegt, bevorzugt von -20 °C bis + 50 °C, weiter bevorzugt im Bereich von -5 bis + 50 °C, noch weiter bevorzugt im Bereich von -5 °C bis +30 °C, besonders bevorzugt im Bereich von 0 °C bis +30 °C, ganz besonders bevorzugt im Bereich von 0 °C bis +10 °C
und/oder
- die flüssige Phase ein oder mehrere Verdünnungsmittel umfasst, wobei das oder die Verdünnungsmittel bevorzugt ausgewählt sind aus der Gruppe bestehend aus Wasser, Alkoholen mit 1 bis 4 Kohlenstoffatomen, Ethern ausgewählt aus der Gruppe bestehend aus aliphatischen Ethern, oligomeren terminal hydroxyfunktionalisierten Ethern und cyclischen Ethern, und deren Mischungen.

## Claims

1. Method for producing 1,2-pentanediol, comprising the step
- reacting a starting material comprising one or both compounds of the group consisting of furfuryl alcohol and furfural in the presence of a first heterogeneous catalyst with hydrogen to form a mixture comprising 1,2-pentanediol and optionally one or more compounds of the group consisting of 1-hydroxy-2-pentanone, furfuryl alcohol and furfural, wherein the hydrogen partial pressure during the reaction in the presence of the first heterogeneous catalyst is in a range of from 0.1 to 0.8 MPa (1 to 8 bar),
wherein the first heterogeneous catalyst comprises:
(i) one or more metals selected from the group consisting of platinum, rhodium, nickel and iridium in metallic form and/or one or more compounds of metals selected from the group consisting of platinum, rhodium, nickel and iridium;
as well as
(ii) one or more support materials.

2. Method according to claim 1, wherein the first heterogeneous catalyst comprises (i) platinum in metallic form and/or one or more platinum(IV)-compounds, wherein the first heterogeneous catalyst is preferably selected from the group consisting of
- catalysts comprising (i) platinum in metallic form and (ii) activated carbon, particularly platinum on activated carbon,
- catalysts comprising (i) platinum in metallic form and (ii) aluminium oxide, particularly platinum on aluminium oxide,
- catalysts comprising (i) platinum in metallic form and (ii) silicon dioxide, particularly platinum on silicon dioxide,
- catalysts comprising (i) platinum in metallic form and (ii) silicon carbide, particularly platinum on silicon carbide,
- catalysts comprising (i) platinum(IV)-oxide and (ii) aluminium oxide, particularly platinum dioxide on aluminium oxide.

3. Method according to one of the previous claims, wherein
- the total concentration of (i) platinum, rhodium, nickel and iridium in metallic form and in form of platinum, rhodium, nickel and iridium contained in compounds of these metals in the first heterogeneous catalyst is in a range of from 0.1 wt.-% to 50 wt.-%, preferably in a range of from 0.5 wt.-% to 20 wt.-%, further preferably in a range of from 1 wt.-% to 10 wt.-%, particularly preferably in a range of from 1 wt.-% to 5 wt.-%, related to the total weight of all components of the first heterogeneous catalyst
and/or
- the amount of the first heterogeneous catalyst is in a range of from 0.1 to 20 wt.-%, preferably in a range of from 0.25 to 15 wt.-%, further preferably in a range of from 0.5 to 12 wt.-%, related to the used amount of furfuryl alcohol, wherein the first heterogeneous catalyst contains (i) platinum in metallic form or one or more compounds of platinum
and/or
- the total concentration of (i) platinum, rhodium, nickel and iridium in metallic form and in form of platinum, rhodium, nickel and iridium contained in compounds of these metals in the first heterogeneous catalyst is in a range of from 0.01 to 10 Mol.-%, preferably in a range of from 0.05 to 5 Mol.%, further preferably in a range of from 0.1 to 2 Mol.-%, related to the used total amount of furfuryl alcohol and furfural.

4. Method according to one of the previous claims, wherein the hydrogen partial pressure during the reaction in the presence of the first heterogeneous catalyst is in a range of from 0.1 to 0.4 MPa (1 to 4 bar).

5. Method according to one of the previous claims, wherein the molar ratio of hydrogen and the total amount of furfuryl alcohol and furfural in the reaction in presence of the first heterogeneous catalyst is 1:1 or more, preferably is in the range of from 4:1 to 100:1, particularly in the range of from 5:1 to 20:1.

6. Method according to one of the previous claims, wherein the reaction in presence of the first heterogeneous catalyst is performed in a gas phase, preferably at a temperature in the range of from 100 °C to 250 °C, further preferably in the range of from 150 °C to 240 °C, particularly preferably in the range of from 170 °C to 230 °C.

7. Method according to claim 6, wherein the reaction in presence of the first heterogeneous catalyst is performed in a reactor which is continuously flown through by a gas flow which comprises said starting material, hydrogen and optionally an inert gas at the entry of the reactor,
wherein
- the flow rate of said gas flow (gas hourly space velocity GHSV) is 500 h⁻¹ to 5000 h⁻¹, particularly preferably 900 h⁻¹ to 3600 h⁻¹, related to the volume of the first heterogeneous catalyst
and/or
- the total concentration of furfuryl alcohol and furfural in the gas flow entering the reactor is 1 Mol-% to 15 Mol-%, preferably 3 Mol-% to 10 Mol-%.

8. Method according to one of claims 1 to 5, wherein
- the reaction is performed in the presence of the first heterogeneous catalyst in a liquid phase
and/or
- at a temperature in the range of from -20 °C to +100 °C, preferably of from -20 °C to +50 °C, further preferably in the range of from -5 to +50 °C, more preferably in the range of from -5 °C to +30 °C, particularly preferably in the range of from 0 °C to +30 °C, especially preferably in the range of form 0 °C to +10 °C.

9. Method according to claim 8, wherein the liquid phase comprises one or more, preferably organic, diluting agents with a pKs-value at 25 °C of higher than or equal 6, preferably with a pKs-value at 25 °C of higher than or equal 8, further preferably with a pKs-value at 25 °C of higher than or equal 10, particularly preferably with a pKs-value at 25 °C of higher than or equal 12,
wherein preferably
- the diluting agent comprises or consist of one or more alcohols with 1 to 4 carbon atoms, preferably selected from the group consisting of methanol, ethanol, n-propanol, isopropanol and mixtures thereof
and/or
- the total amount of diluting agents is in the range of from 25 to 1000 wt.-%, preferably in the range of from 50 to 500 wt.-%, further preferably in the range of from 100 to 300 wt.-%, related to the used total amount of furfuryl alcohol or the total amount of diluting agents is in the range of from 25 to 1000 wt.-%, preferably in the range of from 50 to 500 wt.-%, further preferably in the range of from 100 to 300 wt.-%, related to the used total amount of furfuryl alcohol and furfural.

10. Method according to one of the previous claims, comprising the following steps:
(a) providing a starting material comprising one or both compounds of the group consisting of furfuryl alcohol and furfural;
(b) providing a first heterogeneous catalyst as defined in claim 1;
(c) optionally: providing one or more diluting agents;
(d) producing a mixture comprising the components provided in steps (a) and (b) as well as optionally (c) and contacting said mixture with hydrogen
or
(d') producing a mixture comprising the starting material provided in step (a) and hydrogen as well as optionally an inert gas and contacting said mixture with the first heterogeneous catalyst provided according to step (b);
(e) reacting the starting material in the mixture produced in step (d) or, respectively, (d') with hydrogen in the presence of the first heterogeneous catalyst to form a mixture comprising 1,2-pentanediol and optionally one or more compounds of the group consisting of 1-hydroxy-2-pentanone, furfuryl alcohol and furfural;
(f) optionally: separation of 1,2-pentanediol by distillation.

11. Method according to one of the previous claims, further comprising the step
- reaction of compounds of the group consisting of 1-hydroxy-2-pentanone, furfuryl alcohol and furfural of the mixture produced in a method according to one of the previous claims in the presence of a second heterogeneous catalyst with hydrogen to form 1,2-pentanediol,
wherein the second heterogeneous catalyst comprises
(i') one or more metals selected from the group consisting of platinum, rhodium, ruthenium, nickel, palladium and iridium in metallicform and/or one or more compounds of metals selected from the group consisting of platinum, rhodium, ruthenium, nickel, palladium and iridium;
as well as
(ii') one or more support materials.

12. Method according to one of the previous claims, wherein the support material (ii) of the first heterogeneous catalyst and/or the support material (ii') of the second heterogeneous catalyst is solid at 25 °C and 0.1013 MPa (1013 mbar), preferably also at 230 °C and 0.1013 MPa (1013 mbar), wherein preferably the support material (ii) and/or the support material (ii') is selected form the group consisting of activated carbon, silica, silicon dioxide, silicon carbide, aluminium oxide, zirconium dioxide, titan dioxide, niobium trioxide, cerium dioxide and the mixtures thereof.

13. Method according to one of claims 11 and 12, wherein
- the first and the second heterogeneous catalyst have the same composition,
and/or
- the total concentration of (i) platinum, rhodium, ruthenium, nickel, palladium and iridium in metallic form and in form of platinum, rhodium, ruthenium, nickel, palladium and iridium contained in compounds of these metals in the second heterogeneous catalyst is in a range of from 0.1 wt.-% to 20 wt.-%, preferably in a range of from 0.5 wt.-% to 10 wt.-%, related to the total weight of all components of the second heterogeneous catalyst.

14. Method according to one of the claims 11 to 13, wherein the reaction in the presence of the second heterogeneous catalyst is performed in a gas phase, preferably at a temperature in the range of from 25 °C to 240 °C, particularly preferably in the range of from 100 °C to 130 °C,
wherein preferably the reaction in the presence of the second heterogeneous catalyst is preferably performed in a reactor which is continuously flown through by a gas flow, wherein said gas flow comprises the mixture produced in a method according to one of the claims 1 to 10 as well as hydrogen and optionally an inert gas at the entry of the reactor, wherein
- the flow rate of said gas flow (gas hourly space velocity GHSV) is 500 h⁻¹ to 5000 h⁻¹, particularly preferably 900 h⁻¹ to 3600 h⁻¹, related to the volume of the second heterogeneous catalyst
and/or
- the total concentration of 1-hydroxy-2-pentanone, furfuryl alcohol and furfural in the gas flow entering the reactor is 1 Mol-% to 15 Mol-%, preferably 3 Mol-% to 10 Mol-%.

15. Method according to one of the claims 11 to 13, wherein the reaction in the presence of the second heterogeneous catalyst is performed in a liquid phase wherein preferably
- the temperature is in a range of from -20 °C to +150 °C, preferably of from -20 °C to +50 °C, further preferably in a range of from -5 to +50 °C, more preferably in the range of from -5 °C to +30 °C, particularly preferably in the range of from 0 °C to +30 °C, especially preferably in the range of form 0 °C to+10 °C
and/or
- the liquid phase comprises one or more diluting agents, wherein the diluting agent(s) is/are preferably selected from the group consisting of water, alcohols with 1 to 4 carbon atoms, ethers selected from the group consisting of aliphatic ethers, oligomeric terminally hydroxyfunctionalised ethers and cyclic ethers, and the mixtures thereof.

## Revendications

1. Procédé de préparation de pentane-1,2-diol comprenant l'étape consistant à
- faire réagir un éduit comprenant un ou les deux composés choisis dans le groupe constitué par l'alcool furfurylique et le furfural en présence d'un premier catalyseur hétérogène avec de l'hydrogène, avec formation d'un mélange contenant du pentane-1,2-diol et, le cas échéant, un ou plusieurs composés choisis dans le groupe constitué par le 1-hydroxy-2-pentanone, l'alcool furfurylique et le furfural, dans lequel, dans la réaction en présence du premier catalyseur hétérogène, la pression partielle d'hydrogène se situe dans la gamme allant de 0,1 à 0,8 MPa (de 1 à 8 bars),
dans lequel le premier catalyseur hétérogène comprend
(i) un ou plusieurs métaux choisis dans le groupe constitué par le platine, le rhodium, le nickel et l'iridium sous forme métallique et/ou un ou plusieurs composés de métaux choisis dans le groupe constitué par le platine, le rhodium, le nickel et l'iridium,
ainsi que
(ii) une ou plusieurs matières supports.

2. Procédé selon la revendication 1, dans lequel le premier catalyseur hétérogène comprend (i) du platine sous forme métallique et/ou un ou plusieurs composés de platine (IV),
dans lequel le premier catalyseur hétérogène est choisi de préférence dans le groupe constitué par
- les catalyseurs comprenant (i) du platine sous forme métallique et (ii) du charbon actif, en particulier du platine sur du charbon actif,
- les catalyseurs comprenant (i) du platine sous forme métallique et (ii) de l'alumine, en particulier du platine sur de l'alumine,
- les catalyseurs comprenant (i) du platine sous forme métallique et (ii) du dioxyde de silicium, en particulier du platine sur du dioxyde de silicium,
- les catalyseurs comprenant (i) du platine sous forme métallique et (ii) du carbure de silicium, en particulier du platine sur du carbure de silicium
- les catalyseurs comprenant (i) de l'oxyde de platine (IV) et (ii) de l'alumine, en particulier du dioxyde de platine sur de l'alumine.

3. Procédé selon l'une quelconque des revendications précédentes, dans lequel
- dans le premier catalyseur hétérogène, la concentration totale de (i) platine, rhodium, nickel et iridium sous forme métallique et sous forme de platine, de rhodium, de nickel et d'iridium contenus dans des composés de ces métaux, se situe dans la gamme allant de 0,1 % en poids à 50 % en poids, de préférence de 0,5 % en poids à 20 % en poids, encore de préférence de 1 à 10 % en poids, de manière particulièrement préférée de 1 % en poids à 5 % en poids, par rapport au poids total de tous les composants du premier catalyseur hétérogène
et/ou
- la quantité de premier catalyseur hétérogène se situe dans la gamme allant de 0,1 à 20 % en poids, de préférence dans la gamme allant de 0,25 à 15 % en poids, encore de préférence dans la gamme allant de 0,5 à 12 % en poids, par rapport à la quantité utilisée d'alcool furfurylique, dans lequel le premier catalyseur hétérogène contient (i) du platine sous forme métallique ou un ou plusieurs composés de platine
et/ou
- la concentration totale de (i) platine, rhodium, nickel et iridium sous forme métallique et sous forme de platine, de rhodium, de nickel et d'iridium contenus dans des composés de ces métaux, dans le premier catalyseur hétérogène, se situe dans la gamme allant de 0,01 à 10 % molaire, de préférence dans la gamme allant de 0,05 à 5 % molaire, encore de préférence dans la gamme allant de 0,1 à 2 % molaire, par rapport à la quantité de matière totale utilisée d'alcool furfurylique et de furfural.

4. Procédé selon l'une quelconque des revendications précédentes, dans lequel, dans la réaction en présence du premier catalyseur hétérogène, la pression partielle d'hydrogène se situe dans la gamme allant de 0,1 à 0,4 MPa (de 1 à 4 bars).

5. Procédé selon l'une quelconque des revendications précédentes, dans lequel, dans la réaction en présence du premier catalyseur hétérogène, le rapport molaire entre l'hydrogène et la quantité totale d'alcool furfurylique et de furfural est de 1 : 1 ou plus et se situe de préférence dans la gamme allant de 4 : 1 à 100 : 1, de manière particulièrement préférée dans la gamme allant de 5 : 1 à 20 : 1.

6. Procédé selon l'une quelconque des revendications précédentes, dans lequel la réaction en présence du premier catalyseur hétérogène a lieu en une phase gazeuse, de préférence à une température se situant dans la gamme allant de 100 °C à 250 °C, encore de préférence dans la gamme allant de 150 °C à 240 °C, de manière particulièrement préférée dans la gamme allant de 170 °C à 230 °C.

7. Procédé selon la revendication 6, dans lequel la réaction en présence du premier catalyseur hétérogène a lieu dans un réacteur qui est traversé en continu par un courant de gaz qui, lorsqu'il entre dans le réacteur, comprend ledit éduit, de l'hydrogène et, en option, un gaz inerte,
dans lequel
- le débit de ce courant de gaz par rapport au volume du premier catalyseur hétérogène (Gas hourly space velocity GHSV) est compris entre 500 h⁻¹ et 5000 h⁻¹, de manière particulièrement préférée entre 900 h⁻¹ et 3600 h⁻¹
et/ou
- la concentration totale d'alcool furfurylique et de furfural dans le flux de gaz entrant dans le réacteur est comprise entre 1 % molaire et 15 % molaire, de préférence entre 3 % molaire et 10 % molaire.

8. Procédé selon l'une quelconque des revendications 1 à 5, dans lequel
- la réaction en présence du premier catalyseur hétérogène a lieu en une phase liquide
et/ou
- à une température se situant dans la gamme allant de -20 °C à +100 °C, de préférence de -20 °C à +50 °C, encore de préférence dans la gamme allant de -5 à +50 °C, encore plus préférablement dans la gamme allant de -5 °C à +30 °C, de manière particulièrement préférée dans la gamme allant de 0 °C à +30 °C, de manière tout particulièrement préférée dans la gamme allant de 0 °C à + 10 °C.

9. Procédé selon la revendication 8, dans lequel la phase liquide comprend un ou plusieurs diluants, de préférence organiques, ayant une valeur pKₛ à 25 °C supérieure ou égale à 6, de préférence une valeur pKₛ à 25 °C supérieure ou égale à 8, encore de préférence une valeur pKₛ à 25 °C supérieure ou égale à 10, de manière particulièrement préférée une valeur pKₛ à 25 °C supérieure ou égale à 12 dans lequel, de préférence,
- le diluant comprend ou consiste en un ou plusieurs alcools ayant 1 à 4 atomes de carbone, de préférence choisis dans le groupe constitué par le méthanol, l'éthanol, le n-propanol, l'isopropanol et leurs mélanges,
et/ou
- la quantité totale des diluants se situe dans la gamme allant de 25 à 1000 % en poids, de préférence dans la gamme allant de 50 à 500 % en poids, encore de préférence dans la gamme allant de 100 à 300 % en poids, par rapport à la quantité totale utilisée d'alcool furfurylique, ou la quantité totale des diluants se situe dans la gamme allant de 25 à 1000 % en poids, de préférence dans la gamme allant de 50 à 500 % en poids, encore de préférence dans la gamme allant de 100 à 300 % en poids, par rapport à la quantité totale utilisée d'alcool furfurylique et de furfural.

10. Procédé selon l'une quelconque des revendications précédentes, comprenant les étapes suivantes consistant à:
(a) fournir un éduit comprenant un ou les deux composés choisis dans le groupe constitué par l'alcool furfurylique et le furfural,
(b) fournir un premier catalyseur hétérogène tel que défini dans la revendication 1,
(c) fournir, en option, un ou plusieurs diluants,
(d) préparer un mélange comprenant les composants fournis dans les étapes (a) et (b) ainsi que, en option, (c) et mettre ce mélange en contact avec de l'hydrogène,
ou
(d') préparer un mélange comprenant l'éduit fourni dans l'étape (a) et de l'hydrogène ainsi que, en option, un gaz inerte, et mettre ce mélange en contact avec le premier catalyseur hétérogène fourni selon l'étape (b),
(e) faire réagir, dans le mélange préparé aux étapes (d) ou bien (d'), l'éduit avec de l'hydrogène en présence du premier catalyseur hétérogène, avec formation d'un mélange contenant du pentane-1,2-diol et, le cas échéant, un ou plusieurs composés choisis dans le groupe constitué par le 1-hydroxy-2-pentanone, l'alcool furfurylique et le furfural,
(f) en option, séparer par distillation le pentane-1,2-diol.

11. Procédé selon l'une quelconque des revendications précédentes, comprenant en outre l'étape consistant à
- faire réagir des composés choisis dans le groupe constitué par le 1-hydroxy-2-pentanone, l'alcool furfurylique et le furfural provenant du mélange formé dans un procédé selon l'une quelconque des revendications précédentes, en présence d'un deuxième catalyseur hétérogène, avec de l'hydrogène, avec formation de pentane-1,2-diol,
dans lequel le deuxième catalyseur hétérogène comprend
(i') un ou plusieurs métaux choisis dans le groupe constitué par le platine, le rhodium, le ruthénium, le nickel, le palladium et l'iridium sous forme métallique et/ou un ou plusieurs composés de métaux choisis dans le groupe constitué par le platine, le rhodium, le ruthénium, le nickel, le palladium et l'iridium,
ainsi que
(ii') une ou plusieurs matières supports.

12. Procédé selon l'une quelconque des revendications précédentes, dans lequel la matière support (ii) du premier catalyseur hétérogène et/ou la matière support (ii') du deuxième catalyseur hétérogène à 25 °C et à 0,1013 MPa (1013 mbar), de préférence également à 230 °C et 0,1013 MPa (1013 mbar), est solide, dans lequel, de préférence, la matière support (ii) et/ou la matière support (ii') est choisie dans le groupe constitué par le charbon actif, l'acide silicique, le dioxyde de silicium, le carbure de silicium, l'alumine, le dioxyde de zirconium, le dioxyde de titane, le trioxyde de niobium, le dioxyde de cérium et leurs mélanges.

13. Procédé selon l'une quelconque des revendications 11 à 12, dans lequel
- les premier et deuxième catalyseurs hétérogènes présentent la même composition,
et/ou
- dans le deuxième catalyseur hétérogène, la concentration totale de (i) platine, rhodium, ruthénium, nickel, palladium et iridium sous forme métallique et sous forme de platine, de rhodium, de ruthénium, de nickel, de palladium et d'iridium contenus dans des composés de ces métaux, se situe dans la gamme allant de 0,1 % en poids à 20 % en poids, de préférence de 0,5 % en poids à 10 % en poids, par rapport au poids total de tous les composants du deuxième catalyseur hétérogène.

14. Procédé selon l'une quelconque des revendications 11 à 13, dans lequel la réaction en présence du deuxième catalyseur hétérogène a lieu en une phase gazeuse, de préférence à une température se situant dans la gamme allant de 25 °C à 240 °C, de manière particulièrement préférée dans la gamme allant de 100 °C à 130 °C,
dans lequel, de préférence, la réaction en présence du deuxième catalyseur hétérogène a lieu de préférence dans un réacteur qui est traversé en continu par un courant de gaz, dans lequel ce courant de gaz, lorsqu'il entre dans le réacteur, comprend ledit mélange formé dans un procédé selon l'une quelconque des revendications 1 à 10 ainsi que de l'hydrogène et, en option, un gaz inerte, dans lequel
- le débit de ce courant de gaz par rapport au volume du deuxième catalyseur hétérogène (Gas hourly space velocity GHSV) est compris entre 500 h⁻¹ et 5000 h⁻¹, de préférence entre 900 h⁻¹ et 3600 h⁻¹
et/ou
- la concentration totale de 1-hydroxy-2-pentanone, d'alcool furfurylique et de furfural dans le flux de gaz entrant dans le réacteur est comprise entre 1 % molaire et 15 % molaire, de préférence entre 3 % molaire et 10 % molaire.

15. Procédé selon l'une quelconque des revendications 11 à 13, dans lequel la réaction en présence du deuxième catalyseur hétérogène a lieu en une phase liquide
dans lequel, de préférence,
- la température se situe dans la gamme allant de -20 °C à +150 °C, de préférence de -20 °C à +50 °C, encore de préférence dans la gamme allant de -5 à +50 °C, encore plus préférablement dans la gamme allant de -5 °C à +30 °C, de manière particulièrement préférée dans la gamme allant de 0 °C à +30 °C, de manière tout particulièrement préférée dans la gamme allant de 0 °C à + 10°C
et/ou
- la phase liquide comprend un ou plusieurs diluants, le ou les diluants étant de préférence choisis dans le groupe constitué par l'eau, les alcools ayant 1 à 4 atomes de carbone, les éthers choisis dans le groupe constitué par les éthers aliphatiques, les éthers oligomères hydroxy-fonctionnalisés terminalement et les éthers cycliques et leurs mélanges.
